# EUROPÄISCHE PATENTSCHRIFT

(11) **EP 2 147 972 B1**
(45) Veröffentlichungstag und Bekanntmachung des Hinweises auf die Patenterteilung: **04.11.2015**
(21) Anmeldenummer: 09164073.0
(22) Anmeldetag: 29.06.2009
(51) Int. Cl.: C12N 9/10, C12P 13/22, C12N 9/88

(54) **Verfahren zur Herstellung von L-Tryptophan unter Verwendung von verbesserten Stämmen der Familie Enterobacteriaceae**
Method for manufacturing L-tryptophane using improved strains of the enterobacteriaceae family
Procédé de fabrication de tryptophan L en utilisant des souches améliorées de la famille des enterobacteriaceae

(30) Priorität: 11.07.2008 DE 102008040352
(43) Veröffentlichungstag der Anmeldung: 27.01.2010
(73) Patentinhaber: Evonik Degussa GmbH, 45128 Essen (DE)
(72) Erfinder: Rieping, Mechthild, 33619 Bielefeld (DE); Dusch, Nicole, 33824 Werther (DE)

(56) Entgegenhaltungen:
- EP-A- 0 077 196
- EP-A- 0 745 671
- HU CHANGYUN ET AL: "Mutation analysis of the feedback inhibition site of phenylalanine-sensitive 3-deoxy-D-arabino-heptulosonate 7-phosphate synthase of Escherichia coli." JOURNAL OF BASIC MICROBIOLOGY, Bd. 43, Nr. 5, 2003, Seiten 399-406, XP002545783 ISSN: 0233-111X

### SEQUENZPROTOKOLL

<110> Evonik Degussa GmbH
<120> Verfahren zur Herstellung von L-Aminosäuren unter Verwendung von verbesserten Stämmen der Familie Enterobacteriaceae
<130> 200800207
<160> 10
<170> Patent In version 3.3
<210> 1
   <211> 1053
   <212> DNA
   <213> Escherichia coli
<220>
   <221> CDS
   <222> (1)..(1053)
   <223> aroG Kodierregion
<400> 1
   atg aat tat cag aac gac gat tta cgc atc aaa gaa atc aaa gag tta        48
   Met Asn Tyr Gln Asn Asp Asp Leu Arg Ile Lys Glu Ile Lys Glu Leu
   1               5                   10                  15
   ctt cct cct gtc gca ttg ctg gaa aaa ttc ccc gct act gaa aat gcc        96
   Leu Pro Pro Val Ala Leu Leu Glu Lys Phe Pro Ala Thr Glu Asn Ala
               20                  25                  30
   gcg aat acg gtt gcc cat gcc cga aaa gcg atc cat aag atc ctg aaa       144
   Ala Asn Thr Val Ala His Ala Arg Lys Ala Ile His Lys Ile Leu Lys
           35                  40                  45
   ggt aat gat gat cgc ctg ttg gtt gtg att ggc cca tgc tca att cat       192
   Gly Asn Asp Asp Arg Leu Leu Val Val Ile Gly Pro Cys Ser Ile His
       50                  55                  60
   gat cct gtc gcg gca aaa gag tat gcc act cgc ttg ctg gcg ctg cgt       240
   Asp Pro Val Ala Ala Lys Glu Tyr Ala Thr Arg Leu Leu Ala Leu Arg
   65                  70                  75                  80
   gaa gag ctg aaa gat gag ctg gaa atc gta atg cgc gtc tat ttt gaa       288
   Glu Glu Leu Lys Asp Glu Leu Glu Ile Val Met Arg Val Tyr Phe Glu
                   85                  90                  95
   aag ccg cgt acc acg gtg ggc tgg aaa ggg ctg att aac gat ccg cat       336
   Lys Pro Arg Thr Thr Val Gly Trp Lys Gly Leu Ile Asn Asp Pro His
               100                 105                 110
   atg gat aat agc ttc cag atc aac gac ggt ctg cgt ata gcc cgt aaa       384
   Met Asp Asn Ser Phe Gln Ile Asn Asp Gly Leu Arg Ile Ala Arg Lys
           115                 120                 125
   ttg ctg ctt gat att aac gac agc ggt ctg cca gcg gca ggt gag ttt       432
   Leu Leu Leu Asp Ile Asn Asp Ser Gly Leu Pro Ala Ala Gly Glu Phe
       130                 135                 140
   ctc gat atg atc acc cca caa tat ctc gct gac ctg atg agc tgg ggc       480
   Leu Asp Met Ile Thr Pro Gln Tyr Leu Ala Asp Leu Met Ser Trp Gly
   145                 150                 155                 160
   gca att ggc gca cgt acc acc gaa tcg cag gtg cac cgc gaa ctg gca      528
   Ala Ile Gly Ala Arg Thr Thr Glu Ser Gln Val His Arg Glu Leu Ala
                   165                 170                 175
   tca ggg ctt tct tgt ccg gtc ggc ttc aaa aat ggc acc gac ggt acg      576
   Ser Gly Leu Ser Cys Pro Val Gly Phe Lys Asn Gly Thr Asp Gly Thr
               180                 185                 190
   att aaa gtg gct atc gat gcc att aat gcc gcc ggt gcg ccg cac tgc      624
   Ile Lys Val Ala Ile Asp Ala Ile Asn Ala Ala Gly Ala Pro His Cys
           195                 200                 205
   ttc ctg tcc gta acg aaa tgg ggg cat tcg gcg att gtg aat acc agc      672
   Phe Leu Ser Val Thr Lys Trp Gly His Ser Ala Ile Val Asn Thr Ser
       210                 215                 220
   ggt aac ggc gat tgc cat atc att ctg cgc ggc ggt aaa gag cct aac      720
   Gly Asn Gly Asp Cys His Ile Ile Leu Arg Gly Gly Lys Glu Pro Asn
   225                 230                 235                 240
   tac agc gcg aag cac gtt gct gaa gtg aaa gaa ggg ctg aac aaa gca      768
   Tyr Ser Ala Lys His Val Ala Glu Val Lys Glu Gly Leu Asn Lys Ala
                   245                 250                 255
   ggc ctg cca gca cag gtg atg atc gat ttc agc cat gct aac tcg tcc      816
   Gly Leu Pro Ala Gln Val Met Ile Asp Phe Ser His Ala Asn Ser Ser
               260                 265                 270
   aaa caa ttc aaa aag cag atg gat gtt tgt gct gac gtt tgc cag cag      864
   Lys Gln Phe Lys Lys Gln Met Asp Val Cys Ala Asp Val Cys Gln Gln
           275                 280                 285
   att gcc ggt ggc gaa aag gcc att att ggc gtg atg gtg gaa agc cat      912
   Ile Ala Gly Gly Glu Lys Ala Ile Ile Gly Val Met Val Glu Ser His
       290                 295                 300
   ctg gtg gaa ggc aat cag agc ctc gag agc ggg gag ccg ctg gcc tac      960
   Leu Val Glu Gly Asn Gln Ser Leu Glu Ser Gly Glu Pro Leu Ala Tyr
   305                 310                 315                 320
   ggt aag agc atc acc gat gcc tgc atc ggc tgg gaa gat acc gat gct     1008
   Gly Lys Ser Ile Thr Asp Ala Cys Ile Gly Trp Glu Asp Thr Asp Ala
                   325                 330                 335
   ctg tta cgt caa ctg gcg aat gca gta aaa gcg cgt cgc ggg taa         1053
   Leu Leu Arg Gln Leu Ala Asn Ala Val Lys Ala Arg Arg Gly
               340                 345                 350
<210> 2
   <211> 350
   <212> PRT
   <213> Escherichia coli
<400> 2
   Met Asn Tyr Gln Asn Asp Asp Leu Arg Ile Lys Glu Ile Lys Glu Leu
   1               5                   10                  15
   Leu Pro Pro Val Ala Leu Leu Glu Lys Phe Pro Ala Thr Glu Asn Ala
               20                  25                  30
   Ala Asn Thr Val Ala His Ala Arg Lys Ala Ile His Lys Ile Leu Lys
               35                  40                  45
   Gly Asn Asp Asp Arg Leu Leu Val Val Ile Gly Pro Cys Ser Ile His
       50                  55                  60
   Asp Pro Val Ala Ala Lys Glu Tyr Ala Thr Arg Leu Leu Ala Leu Arg
   65                  70                  75                  80
   Glu Glu Leu Lys Asp Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Ser Val Thr Lys Trp Gly His Ser Ala Ile Val Asn Thr Ser
       210                 215                 220
   Gly Asn Gly Asp Cys His Ile Ile Leu Arg Gly Gly Lys Glu Pro Asn
   225                 230                 235                 240
   Tyr Ser Ala Lys His Val Ala Glu Val Lys Glu Gly Leu Asn Lys Ala
                   245                 250                 255
   Gly Leu Pro Ala Gln Val Met Ile Asp Phe Ser His Ala Asn Ser Ser
               260                 265                 270
   Lys Gln Phe Lys Lys Gln Met Asp Val Cys Ala Asp Val Cys Gln Gln
           275                 280                 285
   Ile Ala Gly Gly Glu Lys Ala Ile Ile Gly Val Met Val Glu Ser His
       290                 295                 300
   Leu Val Glu Gly Asn Gln Ser Leu Glu Ser Gly Glu Pro Leu Ala Tyr
   305                 310                 315                 320
   Gly Lys Ser Ile Thr Asp Ala Cys Ile Gly Trp Glu Asp Thr Asp Ala
                   325                 330                 335
   Leu Leu Arg Gln Leu Ala Asn Ala Val Lys Ala Arg Arg Gly
               340                 345                 350
<210> 3
   <211> 1053
   <212> DNA
   <213> Salmonella typhimurium
<220>
   <221> CDS
   <222> (1)..(1053)
   <223> aroG Kodierregion
<400> 3
   atg aat tat cag aac gac gat tta cgc att aaa gaa atc aac gag tta       48
   Met Asn Tyr Gln Asn Asp Asp Leu Arg Ile Lys Glu Ile Asn Glu Leu
   1               5                   10                  15
   tta cct ccg gtc gcg ctg ctg gaa aag ttt ccc gcc acg gaa aat gca       96
   Leu Pro Pro Val Ala Leu Leu Glu Lys Phe Pro Ala Thr Glu Asn Ala
               20                  25                  30
   gca aat acc gtt gct cac gcg cgc aaa gcc atc cat aaa att ctc aaa      144
   Ala Asn Thr Val Ala His Ala Arg Lys Ala Ile His Lys Ile Leu Lys
           35                  40                  45
   ggc aat gac gat cgt ctg ctg gtg gtg atc ggt cct tgt tca att cat      192
   Gly Asn Asp Asp Arg Leu Leu Val Val Ile Gly Pro Cys Ser Ile His
       50                  55                  60
   gat ccg gca gcg gcg aaa gag tat gcc gcc cgt ttg ctg gcg cta cgc      240
   Asp Pro Ala Ala Ala Lys Glu Tyr Ala Ala Arg Leu Leu Ala Leu Arg
   65                  70                  75                  80
   gat gag ctt caa ggc gag ctt gaa att gtc atg cgc gtc tat ttt gag      288
   Asp Glu Leu Gln Gly Glu Leu Glu Ile Val Met Arg Val Tyr Phe Glu
                   85                  90                  95
   aaa ccg cgt acc acc gtc ggc tgg aaa ggg ctg att aac gat ccg cac      336
   Lys Pro Arg Thr Thr Val Gly Trp Lys Gly Leu Ile Asn Asp Pro His
               100                 105                 110
   atg gat aac agc ttc cag att aac gac ggt ctg cgt att gcg cgc aaa      384
   Met Asp Asn Ser Phe Gln Ile Asn Asp Gly Leu Arg Ile Ala Arg Lys
           115                 120                 125
   ctg ctg ctg gat att aac gac agc ggc ctg cct gcc gcc ggc gaa ttc      432
   Leu Leu Leu Asp Ile Asn Asp Ser Gly Leu Pro Ala Ala Gly Glu Phe
       130                 135                 140
   ctc gat atg atc acg ccg caa tat ctg gcc gat ctg atg agc tgg ggc      480
   Leu Asp Met Ile Thr Pro Gln Tyr Leu Ala Asp Leu Met Ser Trp Gly
   145                 150                 155                 160
   gcc att ggc gcg cgg act act gaa tcc cag gtt cat cgc gaa ttg gcg      528
   Ala Ile Gly Ala Arg Thr Thr Glu Ser Gln Val His Arg Glu Leu Ala
                   165                 170                 175
   tct ggc ctc tct tgt ccg gtc ggt ttt aaa aat ggt act gat ggc acg      576
   Ser Gly Leu Ser Cys Pro Val Gly Phe Lys Asn Gly Thr Asp Gly Thr
               180                 185                 190
   att aaa gtc gcc att gac gcc atc aac gcc gcc ggc gcg ccg cat tgc      624
   Ile Lys Val Ala Ile Asp Ala Ile Asn Ala Ala Gly Ala Pro His Cys
           195                 200                 205
   ttc ctc tcc gtc act aaa tgg ggt cat tcg gcg att gtg aat acc agc      672
   Phe Leu Ser Val Thr Lys Trp Gly His Ser Ala Ile Val Asn Thr Ser
       210                 215                 220
   ggc aac ggc gac tgc cat atc att ctg cgc ggc ggt aaa gcg cca aac      720
   Gly Asn Gly Asp Cys His Ile Ile Leu Arg Gly Gly Lys Ala Pro Asn
   225                 230                 235                 240
   tat agc gcg cag cat gtt gct gag gtg aaa gaa ggc ctc acc aaa gcg      768
   Tyr Ser Ala Gln His Val Ala Glu Val Lys Glu Gly Leu Thr Lys Ala
                   245                 250                 255
   gga ctg acg ccg cag gtc atg atc gat ttc agc cat gcc aac tcc tgt      816
   Gly Leu Thr Pro Gln Val Met Ile Asp Phe Ser His Ala Asn Ser Cys
               260                 265                 270
   aag caa ttt caa aag cag atg gag gtt tgc gcc gat gtc tgc cag cag      864
   Lys Gln Phe Gln Lys Gln Met Glu Val Cys Ala Asp Val Cys Gln Gln
           275                 280                 285
   ata gcg ggc ggt gaa aaa gcg att att ggc gtg atg gta gag agt cat      912
   Ile Ala Gly Gly Glu Lys Ala Ile Ile Gly Val Met Val Glu Ser His
       290                 295                 300
   ctg gta gaa gga aac cag agt ctg gaa agc ggt cag ccg ctg acc tac      960
   Leu Val Glu Gly Asn Gln Ser Leu Glu Ser Gly Gln Pro Leu Thr Tyr
   305                 310                 315                 320
   ggt aaa agc att act gac gcc tgt att ggc tgg gaa gat acc gat gcg     1008
   Gly Lys Ser Ile Thr Asp Ala Cys Ile Gly Trp Glu Asp Thr Asp Ala
                   325                 330                 335
   ctg ctt cgt cag ttg tcg gca gcg gta aaa gcc cgt cgc ggc taa         1053
   Leu Leu Arg Gln Leu Ser Ala Ala Val Lys Ala Arg Arg Gly
               340                 345                 350
<210> 4
   <211> 350
   <212> PRT
   <213> Salmonella typhimurium
<400> 4
   Met Asn Tyr Gln Asn Asp Asp Leu Arg Ile Lys Glu Ile Asn Glu Leu
   1               5                   10                  15
   Leu Pro Pro Val Ala Leu Leu Glu Lys Phe Pro Ala Thr Glu Asn Ala
               20                  25                  30
   Ala Asn Thr Val Ala His Ala Arg Lys Ala Ile His Lys Ile Leu Lys
           35                  40                  45
   Gly Asn Asp Asp Arg Leu Leu Val Val Ile Gly Pro Cys Ser Ile His
       50                  55                  60
   Asp Pro Ala Ala Ala Lys Glu Tyr Ala Ala Arg Leu Leu Ala Leu Arg
   65                  70                  75                  80
   Asp Glu Leu Gln Gly Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Ser Val Thr Lys Trp Gly His Ser Ala Ile Val Asn Thr Ser
       210                 215                 220
   Gly Asn Gly Asp Cys His Ile Ile Leu Arg Gly Gly Lys Ala Pro Asn
   225                 230                 235                 240
   Tyr Ser Ala Gln His Val Ala Glu Val Lys Glu Gly Leu Thr Lys Ala
                   245                 250                 255
   Gly Leu Thr Pro Gln Val Met Ile Asp Phe Ser His Ala Asn Ser Cys
               260                 265                 270
   Lys Gln Phe Gln Lys Gln Met Glu Val Cys Ala Asp Val Cys Gln Gln
           275                 280                 285
   Ile Ala Gly Gly Glu Lys Ala Ile Ile Gly Val Met Val Glu Ser His
   290 295 300
   Leu Val Glu Gly Asn Gln Ser Leu Glu Ser Gly Gln Pro Leu Thr Tyr
   305 310 315 320
   Gly Lys Ser Ile Thr Asp Ala Cys Ile Gly Trp Glu Asp Thr Asp Ala
   325 330 335
   Leu Leu Arg Gln Leu Ser Ala Ala Val Lys Ala Arg Arg Gly
   340 345 350
<210> 5
   <211> 1053
   <212> DNA
   <213> Shigella flexneri
<220>
   <221> CDS
   <222> (1)..(1053)
   <223> aroG Kodierregion
<400> 5
   atg aat tat cag aac gac gat tta cgc atc aaa gaa atc aaa gag tta        48
   Met Asn Tyr Gln Asn Asp Asp Leu Arg Ile Lys Glu Ile Lys Glu Leu
   1               5                   10                  15
   ctt cct cct gtc gca ttg ctg gaa aaa ttc ccc gca act gaa aat gcc        96
   Leu Pro Pro Val Ala Leu Leu Glu Lys Phe Pro Ala Thr Glu Asn Ala
               20                  25                  30
   gcg aat acg gtt gcc cat gcc cga aaa gcg atc cat aag atc ctg aaa       144
   Ala Asn Thr Val Ala His Ala Arg Lys Ala Ile His Lys Ile Leu Lys
           35                  40                  45
   ggt aat gat gat cgc ctg ttg gtg gtg att ggc ccg tgc tca att cat       192
   Gly Asn Asp Asp Arg Leu Leu Val Val Ile Gly Pro Cys Ser Ile His
       50                  55                  60
   gat cct gtc gcg gct aaa gag tat gcc act cgc ttg ctg gcg ctg cgt       240
   Asp Pro Val Ala Ala Lys Glu Tyr Ala Thr Arg Leu Leu Ala Leu Arg
   65                  70                  75                  80
   gaa gag ctg aaa gat gag ctg gaa atc gta atg cgc gtc tat ttt gaa       288
   Glu Glu Leu Lys Asp Glu Leu Glu Ile Val Met Arg Val Tyr Phe Glu
                   85                  90                  95
   aag ccg cgt acc acg gtg ggc tgg aaa ggg ttg att aac gat ccg cat       336
   Lys Pro Arg Thr Thr Val Gly Trp Lys Gly Leu Ile Asn Asp Pro His
               100                 105                 110
   atg gat aac agc ttc cag atc aac gac ggt ctg cgt ata gcc cgt aaa       384
   Met Asp Asn Ser Phe Gln Ile Asn Asp Gly Leu Arg Ile Ala Arg Lys
           115                 120                 125
   ttg ctg ctt gat att aac gac agc ggt ctg cca gcg gcg ggt gag ttt       432
   Leu Leu Leu Asp Ile Asn Asp Ser Gly Leu Pro Ala Ala Gly Glu Phe
       130                 135                 140
   ctc gat atg atc act cct cag tat ctc gct gac ctg atg agc tgg ggc       480
   Leu Asp Met Ile Thr Pro Gln Tyr Leu Ala Asp Leu Met Ser Trp Gly
   145                 150                 155                 160
   gca att ggt gca cgt acc acg gaa tcg cag gtg cac cgc gaa ctg gcg      528
   Ala Ile Gly Ala Arg Thr Thr Glu Ser Gln Val His Arg Glu Leu Ala
                   165                 170                 175
   tct ggt ctt tct tgt ccg gta ggt ttt aaa aat ggc aca gac ggt acg      576
   Ser Gly Leu Ser Cys Pro Val Gly Phe Lys Asn Gly Thr Asp Gly Thr
               180                 185                 190
   att aaa gtg gct atc gat gcc att aat gcc gcc ggt gcg ccg cac tgc      624
   Ile Lys Val Ala Ile Asp Ala Ile Asn Ala Ala Gly Ala Pro His Cys
           195                 200                 205
   ttc ctg tcc gta act aaa tgg ggg cat tcg gcg att gtg aat acc agt      672
   Phe Leu Ser Val Thr Lys Trp Gly His Ser Ala Ile Val Asn Thr Ser
       210                 215                 220
   ggt aac ggc gat tgc cat atc att ctg cgc ggc ggt aaa gag cct aac      720
   Gly Asn Gly Asp Cys His Ile Ile Leu Arg Gly Gly Lys Glu Pro Asn
   225                 230                 235                 240
   tac agc gcg aag cac gtt gct gaa gtg aaa gaa ggg ctg aac aaa gca      768
   Tyr Ser Ala Lys His Val Ala Glu Val Lys Glu Gly Leu Asn Lys Ala
                   245                 250                 255
   ggt ctg cca gct cag gtg atg atc gat ttc agc cat gct aat tcg tcc      816
   Gly Leu Pro Ala Gln Val Met Ile Asp Phe Ser His Ala Asn Ser Ser
               260                 265                 270
   aaa caa ttc aaa aag cag atg gat gtt tgt gct gac gtt tgc cag cag      864
   Lys Gln Phe Lys Lys Gln Met Asp Val Cys Ala Asp Val Cys Gln Gln
           275                 280                 285
   att gcc ggt ggc gaa aag gcc att att ggc gtg atg gtg gaa agc cat      912
   Ile Ala Gly Gly Glu Lys Ala Ile Ile Gly Val Met Val Glu Ser His
       290                 295                 300
   ctg gtg gaa ggc aat cag agc ctc gat agc ggg gag ccg ctg gcc tac      960
   Leu Val Glu Gly Asn Gln Ser Leu Asp Ser Gly Glu Pro Leu Ala Tyr
   305                 310                 315                 320
   ggt aag agc atc acc gat gcc tgc att ggc tgg gaa gat acc gat gct     1008
   Gly Lys Ser Ile Thr Asp Ala Cys Ile Gly Trp Glu Asp Thr Asp Ala
                   325                 330                 335
   ctg tta cgt caa ctg gtg aat gca gta aaa gcg cgt cgc ggg taa         1053
   Leu Leu Arg Gln Leu Val Asn Ala Val Lys Ala Arg Arg Gly
               340                 345                 350
<210> 6
   <211> 350
   <212> PRT
   <213> Shigella flexneri
<400> 6
   Met Asn Tyr Gln Asn Asp Asp Leu Arg Ile Lys Glu Ile Lys Glu Leu
   1               5                   10                  15
   Leu Pro Pro Val Ala Leu Leu Glu Lys Phe Pro Ala Thr Glu Asn Ala
               20                  25                  30
   Ala Asn Thr Val Ala His Ala Arg Lys Ala Ile His Lys Ile Leu Lys
           35                  40                  45
   Gly Asn Asp Asp Arg Leu Leu Val Val Ile Gly Pro Cys Ser Ile His
       50                  55                  60
   Asp Pro Val Ala Ala Lys Glu Tyr Ala Thr Arg Leu Leu Ala Leu Arg
   65                  70                  75                  80
   Glu Glu Leu Lys Asp Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Ser Val Thr Lys Trp Gly His Ser Ala Ile Val Asn Thr Ser
       210                 215                 220
   Gly Asn Gly Asp Cys His Ile Ile Leu Arg Gly Gly Lys Glu Pro Asn
   225                 230                 235                 240
   Tyr Ser Ala Lys His Val Ala Glu Val Lys Glu Gly Leu Asn Lys Ala
                   245                 250                 255
   Gly Leu Pro Ala Gln Val Met Ile Asp Phe Ser His Ala Asn Ser Ser
               260                 265                 270
   Lys Gln Phe Lys Lys Gln Met Asp Val Cys Ala Asp Val Cys Gln Gln
           275                 280                 285
   Ile Ala Gly Gly Glu Lys Ala Ile Ile Gly Val Met Val Glu Ser His
       290                 295                 300
   Leu Val Glu Gly Asn Gln Ser Leu Asp Ser Gly Glu Pro Leu Ala Tyr
   305                 310                 315                 320
   Gly Lys Ser Ile Thr Asp Ala Cys Ile Gly Trp Glu Asp Thr Asp Ala
                   325                 330                 335
   Leu Leu Arg Gln Leu Val Asn Ala Val Lys Ala Arg Arg Gly
               340                 345                 350
<210> 7
   <211> 1053
   <212> DNA
   <213> Escherichia coli
<220>
   <221> CDS
   <222> (1)..(1053)
   <223> aroG Kodierregion
<220>
   <221> mutation
   <222> (98)..(98)
   <223> c -> t Transition
<220>
   <221> mutation
   <222> (632)..(632)
   <223> c -> t Transition
<400> 7
   atg aat tat cag aac gac gat tta cgc atc aaa gaa atc aaa gag tta       48
   Met Asn Tyr Gln Asn Asp Asp Leu Arg Ile Lys Glu Ile Lys Glu Leu
   1               5                   10                  15
   ctt cct cct gtc gca ttg ctg gaa aaa ttc ccc gct act gaa aat gcc       96
   Leu Pro Pro Val Ala Leu Leu Glu Lys Phe Pro Ala Thr Glu Asn Ala
               20                  25                  30
   gtg aat acg gtt gcc cat gcc cga aaa gcg atc cat aag atc ctg aaa      144
   Val Asn Thr Val Ala His Ala Arg Lys Ala Ile His Lys Ile Leu Lys
           35                  40                  45
   ggt aat gat gat cgc ctg ttg gtt gtg att ggc cca tgc tca att cat      192
   Gly Asn Asp Asp Arg Leu Leu Val Val Ile Gly Pro Cys Ser Ile His
       50                  55                  60
   gat cct gtc gcg gca aaa gag tat gcc act cgc ttg ctg gcg ctg cgt      240
   Asp Pro Val Ala Ala Lys Glu Tyr Ala Thr Arg Leu Leu Ala Leu Arg
   65                  70                  75                  80
   gaa gag ctg aaa gat gag ctg gaa atc gta atg cgc gtc tat ttt gaa      288
   Glu Glu Leu Lys Asp Glu Leu Glu Ile Val Met Arg Val Tyr Phe Glu
                   85                  90                  95
   aag ccg cgt acc acg gtg ggc tgg aaa ggg ctg att aac gat ccg cat      336
   Lys Pro Arg Thr Thr Val Gly Trp Lys Gly Leu Ile Asn Asp Pro His
               100                 105                 110
   atg gat aat agc ttc cag atc aac gac ggt ctg cgt ata gcc cgt aaa      384
   Met Asp Asn Ser Phe Gln Ile Asn Asp Gly Leu Arg Ile Ala Arg Lys
           115                 120                 125
   ttg ctg ctt gat att aac gac agc ggt ctg cca gcg gca ggt gag ttt      432
   Leu Leu Leu Asp Ile Asn Asp Ser Gly Leu Pro Ala Ala Gly Glu Phe
       130                 135                 140
   ctc gat atg atc acc cca caa tat ctc gct gac ctg atg agc tgg ggc      480
   Leu Asp Met Ile Thr Pro Gln Tyr Leu Ala Asp Leu Met Ser Trp Gly
   145                 150                 155                 160
   gca att ggc gca cgt acc acc gaa tcg cag gtg cac cgc gaa ctg gca      528
   Ala Ile Gly Ala Arg Thr Thr Glu Ser Gln Val His Arg Glu Leu Ala
                   165                 170                 175
   tca ggg ctt tct tgt ccg gtc ggc ttc aaa aat ggc acc gac ggt acg      576
   Ser Gly Leu Ser Cys Pro Val Gly Phe Lys Asn Gly Thr Asp Gly Thr
               180                 185                 190
   att aaa gtg gct atc gat gcc att aat gcc gcc ggt gcg ccg cac tgc      624
   Ile Lys Val Ala Ile Asp Ala Ile Asn Ala Ala Gly Ala Pro His Cys
           195                 200                 205
   ttc ctg ttc gta acg aaa tgg ggg cat tcg gcg att gtg aat acc agc      672
   Phe Leu Phe Val Thr Lys Trp Gly His Ser Ala Ile Val Asn Thr Ser
       210                 215                 220
   ggt aac ggc gat tgc cat atc att ctg cgc ggc ggt aaa gag cct aac      720
   Gly Asn Gly Asp Cys His Ile Ile Leu Arg Gly Gly Lys Glu Pro Asn
   225                 230                 235                 240
   tac agc gcg aag cac gtt gct gaa gtg aaa gaa ggg ctg aac aaa gca      768
   Tyr Ser Ala Lys His Val Ala Glu Val Lys Glu Gly Leu Asn Lys Ala
                   245                 250                 255
   ggc ctg cca gca cag gtg atg atc gat ttc agc cat gct aac tcg tcc      816
   Gly Leu Pro Ala Gln Val Met Ile Asp Phe Ser His Ala Asn Ser Ser
               260                 265                 270
   aaa caa ttc aaa aag cag atg gat gtt tgt gct gac gtt tgc cag cag      864
   Lys Gln Phe Lys Lys Gln Met Asp Val Cys Ala Asp Val Cys Gln Gln
           275                 280                 285
   att gcc ggt ggc gaa aag gcc att att ggc gtg atg gtg gaa agc cat      912
   Ile Ala Gly Gly Glu Lys Ala Ile Ile Gly Val Met Val Glu Ser His
       290                 295                 300
   ctg gtg gaa ggc aat cag agc ctc gag agc ggg gag ccg ctg gcc tac      960
   Leu Val Glu Gly Asn Gln Ser Leu Glu Ser Gly Glu Pro Leu Ala Tyr
   305                 310                 315                 320
   ggt aag agc atc acc gat gcc tgc atc ggc tgg gaa gat acc gat gct     1008
   Gly Lys Ser Ile Thr Asp Ala Cys Ile Gly Trp Glu Asp Thr Asp Ala
                   325                 330                 335
   ctg tta cgt caa ctg gcg aat gca gta aaa gcg cgt cgc ggg taa         1053
   Leu Leu Arg Gln Leu Ala Asn Ala Val Lys Ala Arg Arg Gly
               340                 345                 350
<210> 8
   <211> 350
   <212> PRT
   <213> Escherichia coli
<400> 8
   Met Asn Tyr Gln Asn Asp Asp Leu Arg Ile Lys Glu Ile Lys Glu Leu
   1               5                   10                  15
   Leu Pro Pro Val Ala Leu Leu Glu Lys Phe Pro Ala Thr Glu Asn Ala
               20                  25                  30
   Val Asn Thr Val Ala His Ala Arg Lys Ala Ile His Lys Ile Leu Lys
           35                  40                  45
   Gly Asn Asp Asp Arg Leu Leu Val Val Ile Gly Pro Cys Ser Ile His
       50                  55                  60
   Asp Pro Val Ala Ala Lys Glu Tyr Ala Thr Arg Leu Leu Ala Leu Arg
   65                  70                  75                  80
   Glu Glu Leu Lys Asp Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Phe Val Thr Lys Trp Gly His Ser Ala Ile Val Asn Thr Ser
       210                 215                 220
   Gly Asn Gly Asp Cys His Ile Ile Leu Arg Gly Gly Lys Glu Pro Asn
   225                 230                 235                 240
   Tyr Ser Ala Lys His Val Ala Glu Val Lys Glu Gly Leu Asn Lys Ala
                   245                 250                 255
   Gly Leu Pro Ala Gln Val Met Ile Asp Phe Ser His Ala Asn Ser Ser
               260                 265                 270
   Lys Gln Phe Lys Lys Gln Met Asp Val Cys Ala Asp Val Cys Gln Gln
           275                 280                 285
   Ile Ala Gly Gly Glu Lys Ala Ile Ile Gly Val Met Val Glu Ser His
       290                 295                 300
   Leu Val Glu Gly Asn Gln Ser Leu Glu Ser Gly Glu Pro Leu Ala Tyr
   305                 310                 315                 320
   Gly Lys Ser Ile Thr Asp Ala Cys Ile Gly Trp Glu Asp Thr Asp Ala
                   325                 330                 335
   Leu Leu Arg Gln Leu Ala Asn Ala Val Lys Ala Arg Arg Gly
               340                 345                 350
<210> 9
   <211> 2054
   <212> DNA
   <213> Escherichia coli
<220>
   <221> CDS
   <222> (501)..(1553)
   <223> aroG Kodierregion
<400> 9
   ccgttattgt cgacgttatt tggcgcgaca ttttcacggg cgtcaggggc tacctggccc     60
   gcatcagctg cggcgtttgc ctggccgtta ttagtttgcg ctccgctatc ggcagccagt    120
   gcggcaccgc tggcaaggct tagagtggca gtcagaaata atgtggccag ttttgtcatt    180
   ttcataggat gctcctgtta tggtcgttat gtcggataac ctcttccaac agtgcatttg    240
   caggtgaata taaggcattg gtttaagatt tcagccaggt tatgaaacgc agcagagaat    300
   cttgaaataa ttaacaaaca aaggagttac agttagaaat tgtaggagag atctcgtttt    360
   tcgcgacaat ctggcgtttt tcttgctaat tccaggatta atccgttcat agtgtaaaac    420
   cccgtttaca cattctgacg gaagatatag attggaagta ttgcattcac taagataagt    480
   atggcaacac tggaacagac atg aat tat cag aac gac gat tta cgc atc aaa    533
                         Met Asn Tyr Gln Asn Asp Asp Leu Arg Ile Lys
                         1               5                   10
   gaa atc aaa gag tta ctt cct cct gtc gca ttg ctg gaa aaa ttc ccc      581
   Glu Ile Lys Glu Leu Leu Pro Pro Val Ala Leu Leu Glu Lys Phe Pro
               15                  20                  25
   gct act gaa aat gcc gcg aat acg gtt gcc cat gcc cga aaa gcg atc      629
   Ala Thr Glu Asn Ala Ala Asn Thr Val Ala His Ala Arg Lys Ala Ile
           30                  35                  40
   cat aag atc ctg aaa ggt aat gat gat cgc ctg ttg gtt gtg att ggc      677
   His Lys Ile Leu Lys Gly Asn Asp Asp Arg Leu Leu Val Val Ile Gly
       45                  50                  55
   cca tgc tca att cat gat cct gtc gcg gca aaa gag tat gcc act cgc      725
   Pro Cys Ser Ile His Asp Pro Val Ala Ala Lys Glu Tyr Ala Thr Arg
   60                  65                  70                  75
   ttg ctg gcg ctg cgt gaa gag ctg aaa gat gag ctg gaa atc gta atg      773
   Leu Leu Ala Leu Arg Glu Glu Leu Lys Asp Glu Leu Glu Ile Val Met
                   80                  85                  90
   cgc gtc tat ttt gaa aag ccg cgt acc acg gtg ggc tgg aaa ggg ctg      821
   Arg Val Tyr Phe Glu Lys Pro Arg Thr Thr Val Gly Trp Lys Gly Leu
               95                  100                 105
   att aac gat ccg cat atg gat aat agc ttc cag atc aac gac ggt ctg      869
   Ile Asn Asp Pro His Met Asp Asn Ser Phe Gln Ile Asn Asp Gly Leu
           110                 115                 120
   cgt ata gcc cgt aaa ttg ctg ctt gat att aac gac agc ggt ctg cca      917
   Arg Ile Ala Arg Lys Leu Leu Leu Asp Ile Asn Asp Ser Gly Leu Pro
       125                 130                 135
   gcg gca ggt gag ttt ctc gat atg atc acc cca caa tat ctc gct gac      965
   Ala Ala Gly Glu Phe Leu Asp Met Ile Thr Pro Gln Tyr Leu Ala Asp
   140                 145                 150                 155
   ctg atg agc tgg ggc gca att ggc gca cgt acc acc gaa tcg cag gtg     1013
   Leu Met Ser Trp Gly Ala Ile Gly Ala Arg Thr Thr Glu Ser Gln Val
                   160                 165                 170
   cac cgc gaa ctg gca tca ggg ctt tct tgt ccg gtc ggc ttc aaa aat     1061
   His Arg Glu Leu Ala Ser Gly Leu Ser Cys Pro Val Gly Phe Lys Asn
               175                 180                 185
   ggc acc gac ggt acg att aaa gtg gct atc gat gcc att aat gcc gcc     1109
   Gly Thr Asp Gly Thr Ile Lys Val Ala Ile Asp Ala Ile Asn Ala Ala
           190                 195                 200
   ggt gcg ccg cac tgc ttc ctg tcc gta acg aaa tgg ggg cat tcg gcg     1157
   Gly Ala Pro His Cys Phe Leu Ser Val Thr Lys Trp Gly His Ser Ala
       205                 210                 215
   att gtg aat acc agc ggt aac ggc gat tgc cat atc att ctg cgc ggc     1205
   Ile Val Asn Thr Ser Gly Asn Gly Asp Cys His Ile Ile Leu Arg Gly
   220                 225                 230                 235
   ggt aaa gag cct aac tac agc gcg aag cac gtt gct gaa gtg aaa gaa     1253
   Gly Lys Glu Pro Asn Tyr Ser Ala Lys His Val Ala Glu Val Lys Glu
                   240                 245                 250
   ggg ctg aac aaa gca ggc ctg cca gca cag gtg atg atc gat ttc agc     1301
   Gly Leu Asn Lys Ala Gly Leu Pro Ala Gln Val Met Ile Asp Phe Ser
               255                 260                 265
   cat gct aac tcg tcc aaa caa ttc aaa aag cag atg gat gtt tgt gct     1349
   His Ala Asn Ser Ser Lys Gln Phe Lys Lys Gln Met Asp Val Cys Ala
           270                 275                 280
   gac gtt tgc cag cag att gcc ggt ggc gaa aag gcc att att ggc gtg     1397
   Asp Val Cys Gln Gln Ile Ala Gly Gly Glu Lys Ala Ile Ile Gly Val
       285                 290                 295
   atg gtg gaa agc cat ctg gtg gaa ggc aat cag agc ctc gag agc ggg     1445
   Met Val Glu Ser His Leu Val Glu Gly Asn Gln Ser Leu Glu Ser Gly
   300                 305                 310                 315
   gag ccg ctg gcc tac ggt aag agc atc acc gat gcc tgc atc ggc tgg     1493
   Glu Pro Leu Ala Tyr Gly Lys Ser Ile Thr Asp Ala Cys Ile Gly Trp
                   320                 325                 330
   gaa gat acc gat gct ctg tta cgt caa ctg gcg aat gca gta aaa gcg     1541
   Glu Asp Thr Asp Ala Leu Leu Arg Gln Leu Ala Asn Ala Val Lys Ala
               335                 340                 345
   cgt cgc ggg taa ggtttaattg tcggatgcgc cgtcagagtg gcgtatccga         1593
   Arg Arg Gly
           350
   tgaatcacca caggcctgat aagtcgcgca gcgtcgcatc aggcaatgtg ctccattgtt   1653
   agcaacaaaa aagccgactc acttgcagtc ggctttctca ttttaaacga atgacgttta   1713
   cttcgcttta ccctggtttg caaccgccgc tgctttcgct gcgatctcgt cagcattacc   1773
   cagataatag cgtttcagcg gtttgaaatt ctcgtcgaac tcatacacca gcggcacgcc   1833
   agtcgggata ttaagctcaa gaatctcttc ttcgctcatg ttatcaagat atttcaccag   1893
   cgcacgtaaa gagttaccgt gtgcagcgat gatcacgcgc tcaccgctct tcatacgcgg   1953
   cagaatagtt tcattccagt aagggatcac gcggtcaatg gtcagcgcca ggctttccgt   2013
   cagcggcagt tctttctcgc tcagtttcgc gtaacgcgga t                       2054
<210> 10
   <211> 350
   <212> PRT
   <213> Escherichia coli
<400> 10
   Met Asn Tyr Gln Asn Asp Asp Leu Arg Ile Lys Glu Ile Lys Glu Leu
   1               5                   10                  15
   Leu Pro Pro Val Ala Leu Leu Glu Lys Phe Pro Ala Thr Glu Asn Ala
               20                  25                  30
   Ala Asn Thr Val Ala His Ala Arg Lys Ala Ile His Lys Ile Leu Lys
           35                  40                  45
   Gly Asn Asp Asp Arg Leu Leu Val Val Ile Gly Pro Cys Ser Ile His
       50                  55                  60
   Asp Pro Val Ala Ala Lys Glu Tyr Ala Thr Arg Leu Leu Ala Leu Arg
   65                  70                  75                  80
   Glu Glu Leu Lys Asp Glu Leu Glu Ile Val Met Arg Val Tyr Phe Glu
                   85                  90                  95
   Lys Pro Arg Thr Thr Val Gly Trp Lys Gly Leu Ile Asn Asp Pro His
               100                 105                 110
   Met Asp Asn Ser Phe Gln Ile Asn Asp Gly Leu Arg Ile Ala Arg Lys
           115                 120                 125
   Leu Leu Leu Asp Ile Asn Asp Ser Gly Leu Pro Ala Ala Gly Glu Phe
       130                 135                 140
   Leu Asp Met Ile Thr Pro Gln Tyr Leu Ala Asp Leu Met Ser Trp Gly
   145                 150                 155                 160
   Ala Ile Gly Ala Arg Thr Thr Glu Ser Gln Val His Arg Glu Leu Ala
                   165                 170                 175
   Ser Gly Leu Ser Cys Pro Val Gly Phe Lys Asn Gly Thr Asp Gly Thr
               180                 185                 190
   Ile Lys Val Ala Ile Asp Ala Ile Asn Ala Ala Gly Ala Pro His Cys
           195                 200                 205
   Phe Leu Ser Val Thr Lys Trp Gly His Ser Ala Ile Val Asn Thr Ser
       210                 215                 220
   Gly Asn Gly Asp Cys His Ile Ile Leu Arg Gly Gly Lys Glu Pro Asn
   225                 230                 235                 240
   Tyr Ser Ala Lys His Val Ala Glu Val Lys Glu Gly Leu Asn Lys Ala
                   245                 250                 255
   Gly Leu Pro Ala Gln Val Met Ile Asp Phe Ser His Ala Asn Ser Ser
               260                 265                 270
   Lys Gln Phe Lys Lys Gln Met Asp Val Cys Ala Asp Val Cys Gln Gln
           275                 280                 285
   Ile Ala Gly Gly Glu Lys Ala Ile Ile Gly Val Met Val Glu Ser His
       290                 295                 300
   Leu Val Glu Gly Asn Gln Ser Leu Glu Ser Gly Glu Pro Leu Ala Tyr
   305                 310                 315                 320
   Gly Lys Ser Ile Thr Asp Ala Cys Ile Gly Trp Glu Asp Thr Asp Ala
                   325                 330                 335
   Leu Leu Arg Gln Leu Ala Asn Ala Val Lys Ala Arg Arg Gly
               340                 345                 350

## Beschreibung

Die vorliegende Erfindung betrifft feedback-resistente Varianten der 3-Desoxy-D-Arabino-Heptulosonat-7-Phosphat-Synthase (DAHP-Synthase), rekombinante Mikroorganismen der Familie Enterobacteriaceae enthaltend diese Enzyme sowie ihre Verwendung zur fermentativen Herstellung von organisch-chemischen Verbindungen.

### Stand der Technik

Organisch-chemischen Verbindungen, insbesondere aromatische L-Aminosäuren, finden in der Humanmedizin und in der pharmazeutischen Industrie, in der Lebensmittelindustrie und in der Tierernährung Anwendung.

Werden im Folgenden organisch-chemische Verbindungen erwähnt, sind damit eine oder mehrere Verbindungen ausgewählt aus der Gruppe der Verbindungen des Shikimat-Stoffwechselweges, wie die aromatischen L-Aminosäuren, L-Tyrosin, L-Phenylalanin und L-Tryptophan, Chorismat, Enterobactin, Menaquinon, Tetrahydrofolat und Ubiquinon gemeint. Bevorzugt sind die aromatischen L-Aminosäuren, besonders bevorzugt sind L-Tryptophan und L-Phenylalanin.

Es ist bekannt, dass organisch-chemischen Verbindungen durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht organisch-chemischen Verbindungen überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Der Shikimat-Stoffwechselweg zur Synthese von u.a. Chorismat, Enterobactin, Menaquinon, Tetrahydrofolat, Ubiquinon und den aromatischen Aminosäuren ist stark reguliert. Die Aromatenbiosynthese wird in E. coli sowohl auf genetischer Ebene durch Attenuation und Repression wie auch durch Endprodukthemmung von Enzymen (Frost und Draths, Annual review of microbiology, 49:557-79 (1995); Pittard, Genes to Cells 1(8):717-25 (1996)) reguliert. Uber diesen Weg wird die Produktion aromatischer Metabolite genau an den Bedarf der Zelle angepasst. Der Shikimisäureweg wird vor allem durch die Steuerung der Eingangsreaktion, die durch drei verschiedene Isoenzyme der DAHP-Synthase (kodiert durch die Gene aroF, aroG und aroH) katalysiert wird, reguliert. Das AroG-Genprodukt macht in E. coli 80% der Gesamtaktivität aller DAHP-Synthasen aus (Tribe et al., Journal of Bacteriology 127(3): 1085-1097 (1976); Pittard, Genes to Cells 1(8):717-25 (1996)); AroG wird durch die aromatische Aminosäure L-Phenylalanin zu 95% inhibiert. AroF wird durch L-Tyrosin ebenfalls zu 95% inhibiert, während AroH bis zu 60% durch L-Tryptophan inhibiert werden kann (Camakaris und Pittard, Journal of Bacteriology 120(2):590-7 (1974)). Da AroH nur teilweise durch L-Tryptophan inhibiert wird, wird der Shikimisäureweg auch bei hohen Konzentrationen aller drei aromatischen Aminosäuren nicht vollständig inhibiert, so dass durch die verbleibende Restaktivität immer noch etwas Chorisminsäure gebildet werden kann, die dann für andere Biosynthesewege verfügbar ist. Auf genetischer Ebene reprimiert der Regulator TyrR in Anwesenheit von Phenylalanin und Tryptophan die Transkription von aroG (Baseggio et al., Journal of Bacteriology 172(5):2547-57 (1990); Davies et al., Gene 33(3):323-31 (1985)).

Die Nukleotidsequenz der Wildform des für die 3-Desoxy-D-Arabino-Heptulosonat-7-Phosphat-Synthase von Escherichia coli kodierenden Gens aroG wurde von Davies und Davidson (Nucleic Acids Research 10 (13): 4045-4058 (1982)) bestimmt und ist inklusive der stromaufwärts (upstream) und stromabwärts (downstream) liegenden Bereiche in der Datenbank des National Center for Biotechnology Information (NCBI) der National Library of Medicin (Bethesda, MD, USA) unter der Zugangsnummer (accession number) NC000913 (Region: 784.856 -> 785.908 und upstream/downstream) allgemein verfügbar.

In der Patentanmeldung EP 1 270 721 wird die förderliche Wirkung der Verwendung eines aroG-Allels auf die Produktion und Herstellung der aromatischer Aminosäuren L-Phenylalanin und L-Tryptophan mit Stämmen der Gattung Escherichia beschrieben, wobei es sich in diesem Fall um ein Allel handelt, worin das Prolin an Position 150 durch ein Leucin ersetzt ist. In der Patentanmeldung WO2005/056776 werden Allele beschrieben, worin das Valin an Position 57 durch ein Alanin bzw. das Cystein an Position 61 durch Arginin ersetzt ist. Kikuchi et al. (Applied and Environmental Microbiology 63(2):761-762 (1996) beschreiben darüber hinaus Allele mit Mutationen an den Positionen 146, 147 und 202.

Weil die Biosynthese organisch-chemische Verbindungen des Shikimat-Stoffwechselweges sehr komplex ist, vielfältig reguliert wird und darüber hinaus mit verschiedenen anderen Stoffwechselwegen in der Zelle vernetzt ist, ist es wünschenswert, viele Varianten der 3-Desoxy-D-Arabino-Heptulosonat-7-Phosphat-Synthase, die sich in ihrer Hemmbarkeit durch L-Phenylalanin unterscheiden, zur Verfügung zu haben. Durch die Komplexität des Biosyntheseweges ist nicht vorhersagbar, mit welcher Variante eine verbesserte Produktion der organisch-chemische Verbindungen, bevorzugt L-Tryptophan und L-Phenylalanin, erzielt werden kann.

### Aufgabe der Erfindung

Die Erfinder haben sich die Aufgabe gestellt, neue Varianten der 3-Desoxy-D-Arabino-Heptulosonat-7-Phosphat-Synthase zur Verfügung zu stellen, die eine im Vergleich zum Wildtyp (WT) -Enzym erhöhte Feedback-Resistenz hinsichtlich L-Phenylalanin besitzen und somit neue Grundlagen für verbesserte Verfahren zur fermentativen Herstellung von organisch-chemische Verbindungen, bevorzugt L-Tryptophan und L-Phenylalanin, mit Mikroorganismen der Familie Enterobacteriaceae bereitzustellen.

### Beschreibung der Erfindung

Gegenstand der Erfindung sind Allele des aroG-Gens, nämlich Nukleotidsequenzen (DNA), die für feedback-resistente Varianten des Enzyms 3-Desoxy-D-Arabino-Heptulosonat-7-Phosphat-Synthase (DAHP-Synthase AroG) kodieren, dadurch gekennzeichnet, dass deren Aminosäuresequenz die Aminosäuresequenz von SEQ ID NO:2 aufweist, einschließlich Deletion und/oder Insertion von 1 bis 20 Aminosäuren, und im Wesentlichen eine Länge von 350 Aminosäuren umfasst und in den zugehörigen Aminosäuresequenzen die Aminosäurenaustausche in der Aminosäuresequenz von SEQ ID NO:2 ausgewählt aus der Gruppe
a) Austausch des L-Alanin an Position 33 gegen L-Valin,
b) Austausch des L-Alanin an Position 33 gegen L-Valin und Austausch des L-Serin an Position 211 gegen L-Phenylalanin,
enthält und die Aminosäuresequenz von SEQ ID NO:2 gegebenenfalls zusätzlich eine oder mehrere der Austausche, ausgewählt aus der Gruppe
c) Austausch des L-Lysin an Position 14 gegen eine Aminosäure ausgewählt aus der Gruppe L-Asparagin und L-Glutamin, bevorzugt L-Asparagin,
d) Austausch des L-Valin an Position 67 gegen eine Aminosäure ausgewählt aus der Gruppe L-Alanin, L-Lysin, L-Arginin und L-Histidin, bevorzugt L-Alanin,
e) Austausch des L-Threonin an Position 74 gegen eine Aminosäure ausgewählt aus der Gruppe L-Alanin, L-Lysin, L-Arginin und L-Histidin, bevorzugt L-Alanin,
f) Austausch des L-Glutaminsäure an Position 81 gegen eine Aminosäure ausgewählt aus der Gruppe L-Asparaginsäure, L-Serin, L-Alanin und L-Threonin, bevorzugt L-Asparaginsäure,
g) Austausch des L-Lysin an Position 84 gegen eine Aminosäure ausgewählt aus der Gruppe L-Glutamin, L-Serin, L-Threonin, L-Glutaminsäure, L-Asparaginsäure und L-Asparagin, bevorzugt L-Glutamin,
h) Austausch des L-Asparaginsäure an Position 85 gegen eine Aminosäure ausgewählt aus der Gruppe L-Alanin und Glycin, bevorzugt Glycin,
i) Austausch des L-Glutaminsäure an Position 238 gegen eine Aminosäure ausgewählt aus der Gruppe L-Asparaginsäure, L-Serin, L-Alanin und L-Threonin, bevorzugt L-Alanin,
j) Austausch des L-Lysin an Position 244 gegen eine Aminosäure ausgewählt aus der Gruppe L-Glutamin, L-Glutaminsäure, L-Asparaginsäure, L-Serin, L-Threonin und L-Asparagin, bevorzugt L-Glutamin,
k) Austausch des L-Asparagin an Position 254 gegen eine Aminosäure ausgewählt aus der Gruppe L-Threonin, L-Serin, L-Glutaminsäure und L-Asparaginsäure, bevorzugt L-Threonin,
l) Austausch des L-Prolin an Position 259 gegen eine Aminosäure ausgewählt aus der Gruppe L-Serin, L-Threonin, bevorzugt L-Threonin,
m) Austausch des L-Alanin an Position 260 gegen L-Prolin,
n) Austausch des L-Serin an Position 272 gegen eine Aminosäure ausgewählt aus der Gruppe L-Cystein und L-Methionin, bevorzugt L-Cystein,
o) Austausch des L-Lysin an Position 276 gegen eine Aminosäure ausgewählt aus der Gruppe L-Glutamin, L-Glutaminsäure, L-Asparaginsäure, L-Serin, L-Threonin und L-Asparagin, bevorzugt L-Glutamin,
p) Austausch der L-Asparaginsäure an Position 280 gegen eine Aminosäure ausgewählt aus der Gruppe L-Serin, L-Alanin, L-Glutaminsäure und L-Threonin, bevorzugt L-Glutaminsäure,
q) Austausch des L-Glutaminsäure an Position 313 gegen eine Aminosäure ausgewählt aus der Gruppe L-Serin, L-Alanin, L-Asparaginsäure und L-Threonin, bevorzugt L-Asparaginsäure,
r) Austausch des L-Glutaminsäure an Position 316 gegen eine Aminosäure ausgewählt aus der Gruppe L-Glutamin, L-Serin, L-Threonin, L-Asparaginsäure und L-Asparagin, bevorzugt L-Glutamin,
s) Austausch des L-Alanin an Position 319 gegen eine Aminosäure ausgewählt aus der Gruppe L-Threonin, L-Valin und L-Serin, bevorzugt L-Threonin,
t) Austausch des L-Alanin an Position 342 gegen eine Aminosäure ausgewählt aus der Gruppe L-Serin, L-Valin und L-Threonin, bevorzugt L-Serin oder L-Valin,
u) Austausch des L-Asparagin an Position 343 gegen eine Aminosäure ausgewählt aus der Gruppe L-Asparaginsäure, L-Glutaminsäure, L-Serin, L-Alanin und L-Threonin, bevorzugt L-Alanin.
enthält.

Bevorzugt besitzen die kodierten Polypeptide eine Länge entsprechend 350 Aminosäuren.

Bevorzugt sind isolierte Polynukleotide gemäß SEQ ID NO: 1, dadurch gekennzeichnet, dass deren Sequenz folgende Austausche enthält
- an der Position 98 Thymin oder
- an den Positionen 98 und 632 Thymin.

Gegenstand der Erfindung sind weiterhin rekombinante organisch-chemische Verbindungen ausgewählt aus der Gruppe der Verbindungen des Shikimat-Stoffwechselweges, bevorzugt die aromatischen L-Aminosäuren produzierende Mikroorganismen der Familie Enterobacteriaceae, die ein erfindungsgemäßes feedback-resistentes aroG-Allel enthalten, das für die 3-Desoxy-D-Arabino-Heptulosonat-7-Phosphat-Synthase AroG kodiert, und die vermehrt die genannten Verbindungen produzieren und in der Zelle oder im Medium anreichern.

Gegenstand der Erfindung sind bevorzugt rekombinante L-Tryptophan produzierende Mikroorganismen der Familie Enterobacteriaceae, die ein erfindungsgemäßes feedback-resistentes aroG-Allel enthalten, das für die 3-Desoxy-D-Arabino-Heptulosonat-7-Phosphat-Synthase AroG kodiert, und die vermehrt L-Tryptophan produzieren und in der Zelle oder im Medium anreichern.

Gegenstand der Erfindung sind ebenfalls L-Aminosäure produzierende Mikroorganismen der Familie Enterobacteriaceae, die resistent gegen L-Phenylalanin sind und die vermehrt L-Tryptophan produzieren und in der Zelle oder im Medium anreichern. L-Phenylalanin wird im Allgemeinen in Konzentrationen von ≥ (größer/gleich) 2 bis ≤ (kleiner/gleich) 15 mM eingesetzt. Als Ausgangspunkt für den Vergleich dienen jeweils die gegen L-Phenylalanin geringer resistenten Mikroorganismen, an denen die erfindungsgemäße Maßnahme nicht durchgeführt wurde.

Gegenstand der Erfindung ist ebenso ein Verfahren zur fermentativen Herstellung von organisch-chemischen Verbindungen ausgewählt aus der Gruppe der Verbindungen des Shikimat-Stoffwechselweges, bevorzugt der aromatischen L-Aminosäuren, insbesondere L-Tryptophan, unter Verwendung von rekombinanten Mikroorganismen der Familie Enterobacteriaceae, die insbesondere bereits vor dem erfindungsgemäßen Verlust der feedback-Inhibierung die genannten organisch-chemischen Verbindungen produzieren, und in denen mindestens das aroG-Allel oder für dessen Genprodukt kodierende Nukleotidsequenzen exprimiert, bevorzugt verstärkt oder überexprimiert wird bzw. werden.

Zu den vor dem erfindungsgemäßen Verlust der feedback-Inhibierung L-Aminosäure produzierenden Mikroorganismen zählen dabei nicht die Wildtypstämme und häufig benutzten Laborstämme wie unter anderen DH5α, DH5αmcr, W3110, MG1655, MC4100, Y1089, H560 und MM152.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Tryptophan und L-Phenylalanin.

Der Begriff "Expression" beschreibt in diesem Zusammenhang die Realisierung der intrazellulären Aktivität eines oder mehrerer Enzyme oder Proteine in einem Mikroorganismus, die durch die entsprechende exprimierte DNA kodiert werden.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs oder des Allels bzw. der Allele um mindestens eine (1) Kopie erhöht, einen starken Promotor funktionell mit dem Gen verknüpft, die endogene Promotor- und Regulationsregion oder die Ribosomenbindungsstelle, die sich stromaufwärts des Strukturgens befindet, mutiert oder ein Gen oder Allel oder ORF verwendet, das für ein entsprechendes Enzym bzw. Protein mit einer hohen Aktivität kodiert und gegebenenfalls diese Maßnahmen kombiniert.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2): 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Die Kopienzahl des erfindungsgemäßen aroG-Allels wird bevorzugt um höchstens acht (8) Kopien erhöht, besonders bevorzugt um höchstens vier (4) Kopien. Eine solche gemäßigte Expression ist zum Beispiel erreichbar durch die Verwendung von Plasmiden mit lacI^{Q}-Allel-kontrollierten Genen (Glascock und Weickert, Gene 223, 221-231 (1998)) ohne Induktion durch IPTG-Zugabe, welches zu einer sehr schwachen Expression des ohne eigenen Promotor klonierten aroG-Gens führt. Der Transkriptionsstart des aroG-Gens wurde 40 Basenpaare vor dem ATG-Startkodon lokalisiert (Baseggio et al., Journal of Bacteriology 172(5): 2547-2557 (1990)) und nicht zusammen mit der für die DAHP-Synthase kodierenden Nukleotidsequenz auf das Plasmid übertragen.

Zur Erzeugung von aroG-Mutationen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Zur Konstruktion von Mutationen im aroG-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene aroG-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Die erzeugten Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pK03 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten Allele durch Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Tryptophan-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. Es konnte gezeigt werden, dass der tac-Promoter 3 mal stärker als der trp-Promoter und 10 mal stärker als der lac-Promoter ist (de Boer et al., Proceedings of the National Academy of Sciences of the USA 80:21-25 (1983)). In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression von lac-Promotor-Genfusionen kontrollieren (Glascock und Weickert, Gene 223, 221-231 (1998)), die Verwendung dieser Plasmide ohne Induktion durch IPTG-Zugabe führt zu einer sehr schwachen Expression von ohne eigenem Promotor klonierten Genen. Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Eine andere zeitliche Genexpression kann beispielsweise wie bei Walker et al. (Journal of Bacteriology 181: 1269-80 (1999)) beschrieben durch die Verwendung des Wachstumsphasenabhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zweibis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das aroG-Allel oder für dessen Genprodukt kodierende Nukleotidsequenzen trägt.

Unter dem Begriff Transformation versteht man die Aufnahme einer isolierten Nukleinsäure durch einen Wirt (Mikroorganismus).

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verlust der feedback-Inhibierung oder Überexpression durchgeführt wird.

Zur Bestimmung des Ausmaßes der Feedback-Resistenz einer erfindungsgemäßen Variante der 3-Desoxy-D-Arabino-Heptulosonat-7-Phosphat-Synthase wird beispielsweise der Thiobarbiturat-Assay nach Schoner und Herrmann (The Journal of Biological Chemistry 251(18): 5440-5447 (1976); modifiziert nach Waravdekar und Saslaw, Biochimica et Biophysica Acta 24, 439 (1957)) verwendet. Dieser Test beruht auf der Periodat-Oxidation von DAHP zu Formylpyruvat, welches mit Thiobarbiturat zu einem farbigen Komplex reagiert. Eine 100 µl Mischung enthaltend PhosphatPuffer (50mM, pH 7) und 75 µmol Phosphoenolpyruvat (PEP) sowie 0,3 µmol Erythrose-4-Phosphat (E4P) wird 5 min bei 37°C im Wasserbad inkubiert. Durch Zugabe von 50 µl Rohextrakt, gewonnen durch Ultraschallaufschluß (Simpson et al., Journal of Bacteriology 107(3): 798-805 (1971), wird die Reaktion gestartet und nach 10 min durch Zugabe von 300 µl einer 0,6 M Trichloressigsäurelösung gestoppt. Ausgefälltes Protein wird abzentrifugiert. Zu 400 µl des Reaktionsansatzes werden 100 µl der Lösung I (0,2 M Natriumperiodat in 9 M H₃PO₄) gegeben und 20 min bei 25°C im Wasserbad inkubiert. Dazu werden 400 µl der Lösung II (0,75 M Natriumarsenat in 0,5 M Na₂SO₄ und 0,05 M H₂SO₄) gegeben, bei Raumtemperatur 2 min inkubiert, und danach werden 3 ml der Lösung III (0,04 M Thiobarbiturat in 0,5 M Na₂SO₄) zugesetzt und 15 min im kochenden Wasserbad erhitzt. Als Nullprobe wird Wasser verwendet. Die Nullprobe bleibt farblos und die getesteten Rohextrakte färben sich mehr oder weniger intensiv pink; der Gehalt an DAHP wird über die Änderung der Extinktion nach Abkühlung der Lösung bei 549 nm gemessen (Extinktionskoeffizient ε= 3,34 x 10⁴ M⁻¹cm⁻¹). Zur Bestimmung des Ausmaßes der Feedback-Resistenz wird die katalytische Aktivität der DAHP-Synthase Varianten in An- und Abwesenheit von 2 mM L-Phenylalanin gemessen.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Ein Gen oder Allel ist chemisch ein Polynukleotid. Ein anderer Begriff hierfür ist Nukleinsäure, insbesondere Desoxyribonukleinsäure.

Die Begriffe Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Gegenstand der Erfindung ist ein Verfahren zur Herstellung von organisch-chemischen Verbindungen ausgewählt aus der Gruppe der Verbindungen des Shikimat-Stoffwechselweges, bevorzugt der aromatischen L-Aminosäuren, insbesondere L-Tryptophan, durch Fermentation der erfindungsgemäßen rekombinanten Mikroorganismen der Familie Enterobacteriaceae, dadurch gekennzeichnet, dass man
a) die die organisch-chemischen Verbindungen produzierenden Mikroorganismen, in denen man das aroG-Gen oder für dessen Genprodukt kodierende Nukleotidsequenzen oder Allele exprimiert, insbesondere überexprimiert, wobei die Verlust der feedback-Inhibierung durch eine Mutagenesemaßnahme innerhalb des aroG-Gens erzielt wird, in einem Medium kultiviert unter Bedingungen, bei denen die organisch-chemischen Verbindungen im Medium oder in den Zellen angereichert wird, und,
b) die organisch-chemischen Verbindungen isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem exprimierten oder überexprimierten aroG-Allel, die ebenfalls Gegenstand der vorliegenden Erfindung sind, können die organisch-chemischen Verbindungen aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens, Allels oder ORFs verstärkenden Promotor enthält. Bei diesem Promotor handelt es sich um den durch verstärkende Mutation aus der eigenen, upstream des Gens, Allels oder ORFs befindlichen regulatorischen Sequenz hervorgegangenen Promotor.

Zur Herstellung der die organisch-chemischen Verbindungenanreichernden Stämme der Familie Enterbacteriaceae, welche ein aroG-Allel enthalten, werden bevorzugt Stämme verwendet (Ausgangsstämme bzw. Elternstämme), die bereits die Fähigkeit besitzen, die gewünschte organisch-chemische Verbindung in der Zelle anzureichern und/oder in das sie umgebende Nährmedium auszuscheiden bzw. in der Fermentationsbrühe zu akkumulieren. Hierfür kann auch der Ausdruck "produzieren" verwendet werden. Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an L-Aminosäuren, bevorzugt L-Tryptophan und L-Phenylalanin, in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäure-ausscheidenden Stämme produzieren eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Homoserin, L-Leucin, L-Valin, L-Alanin, L-Isoleucin und L-Histidin. Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrere weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phosphoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Bei den der Erfindung zugrunde liegenden Arbeiten wurde gefunden, dass Mikroorganismen der Familie Enterobacteriaceae nach Expression oder Überexpression der aroG-Allele, vermehrt L-Aminosäuren, insbesondere L-Tryptophan, produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella typhimurium und Shigella flexneri ist die Nukleotidsequenz für das aroG-Gen ebenso bekannt (Accession No.: NC_003197 (REGION: 823772-824824) und Accession No.: NC_004337 (REGION: komplementär (571488-572540)). Weitere Nukleotidsequenzen für das aroG-Gen findet man aus folgenden Enterobacteriaceae: Shigella boydii (Accession No.: CP000036); Shigella dysenteriae (Accession No.: CP000034); Shigella sonnei (Accession No.: CP000038); Salmonella enterica (Accession No.: AE017220); Serratia proteamaculans (Accession No.: CP000826).

Das aroG-Gen von Escherichia coli K12 wird unter anderem durch folgende Angaben beschrieben:

| | |
|---|---|
| Bezeichnung: | 3-Desoxy-D-Arabino-Heptulosonat-7-Phosphat-Synthase (DAHP-Synthase) AroG |
| Funktion: | Eisenabhängiges Metalloenzym, welches sowohl durch die genetische Regulation über Attenuation und Repression als auch allosterisch durch L-Phenylalanin reguliert bzw. inhibiert wird. Das Enzym kondensiert Phosphoenolpyruvat (PEP) aus der Glykolyse und Erythrose-4-Phosphat (E4P) aus dem Pentosephosphatweg in der Eingangsreaktion des Aromatenbiosyntheseweges zu DAHP. |
| | |
| Referenz: | Ray et al., Journal of Bacteriology 170(12):5500-6 (1988); |
| | Davies et al., Nucleic Acids Research 10 (13) :4045-8 (1982); |
| | Frost and Draths, Annual review of microbiology, 49:557-79 (1995); |
| | Pittard, Genes to Cells 1(8) :717-25 (1996) |
| | |
| Accession No.: | NC000913 (Region: 784.856 -> 785.908) |
| Das kodierte Polypeptid besitzt eine Länge von 350 Aminosäuren. | |
| Alternativer Genname: | b0754 |

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum aroG-Gen von Escherichia coli unter der SEQ ID No. 1 und die bekannten Sequenzen zum aroG-Gen von Salmonella typhimurium und Shigella flexneri unter den SEQ ID No. 3 und SEQ ID No. 5 dargestellt. Die von diesen Leserahmen kodierten Proteine sind als SEQ ID No. 2, SEQ ID No. 4 und SEQ ID No. 6 dargestellt. Die bekannte Sequenz zum aroG-Gen von Escherichia coli inklusive der stromaufwärts (upstream) und stromabwärts (downstream) gelegenen Nukleotidsequenzen ist unter der SEQ ID No. 9, das von diesem Leserahmen kodierte Protein ist unter SEQ ID No. 10 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Zu den Allelen des aroG-Gens, welche funktionsneutrale Sinnmutationen enthalten, zählen unter anderem solche, die zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Weiterhin kann es für die Produktion von L-Aminosäuren, insbesondere L-Tryptophan, mit Stämmen der Familie Enterobacteriaceae vorteilhaft sein, zusätzlich zur Expression des aroG-Allels, ein oder mehrere Enzym(e) der bekannten Biosynthesewege oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So können beispielsweise für die Produktion von L-Tryptophan, gleichzeitig eines oder mehrere der Gene, ausgewählt aus der Gruppe
- mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5,168,056),
- das für die Transketolase B kodierende tktB-Gen (US5,168,056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA), EP1449918),
verstärkt, insbesondere überexprimiert werden.

Weiterhin kann es für die Produktion von L-Aminosäuren, insbesondere L-Tryptophan, vorteilhaft sein, zusätzlich zur Verlust der feedback-Inhibierung des aroG-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Tryptophan gleichzeitig eines oder mehrere der Gene, ausgewählt aus der Gruppe
- das für die Tryptophanase kodierende tnaA-Gen (US4,371,614),
- das für den Repressor des trp-Operons kodierende trpR-Gen (US4,371,614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),
- das für das Tryptophan spezifische Transportprotein kodierende mtr-Gen (US5,756,345),
- das für die Tryptophan-Permease kodierende tnaB-Gen (US5,756,345),
- das für den Transporter für aromatische Aminosäuren kodierende aroP-Gen (US5,756,345),
- das für die L-Serin Deaminase kodierende sdaA-Gen (EP0149539),
- das für die Glukose-6-Phosphat-Isomerase kodierende pgi-Gen (WO87/01130),
- das für die Tyrosin-Aminotransferase kodierende tyrB-Gen (WO87/01130)
abgeschwächt, gegebenenfalls ausgeschaltet oder die Expression verringert werden. Diese Massnahmen werden gegebenenfalls zusätzlich zu bzw. in geeigneter Kombination mit den angegebenen Massnahmen der Verstärkung von Genen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservativen Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC•GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Das erfindungsgemäße Verfahren dient zur fermentativen Herstellung von organisch-chemische Verbindungen ausgewählt aus der Gruppe der Verbindungen des Shikimat-Stoffwechselweges, bevorzugt sind die aromatischen L-Aminosäuren L-Tyrosin, L-Phenylalanin und L-Tryptophan, Chorismat, Enterobactin, Menaquinon, Tetrahydrofolat und Ubiquinon. Besonders bevorzugt sind L-Tryptophan und L-Phenylalanin.

## Patentansprüche

1. Nukleotidsequenz (DNA), die für eine feedback-resistente Variante des Enzyms 3-Desoxy-D-Arabino-Heptulosonat-7-Phosphat-Synthase (DAHP-Synthase) kodiert, **dadurch gekennzeichnet, dass** deren Aminosäuresequenz die Aminosäuresequenz von SEQ ID NO:2 aufweist, einschließlich Deletion und/oder Insertion von 1 bis 20 Aminosäuren und im Wesentlichen eine Länge von 350 Aminosäuren umfasst und in den zugehörigen Aminosäuresequenzen die Aminosäurenaustausche in der Aminosäuresequenz von SEQ ID NO:2, ausgewählt aus der Gruppe
a) Austausch des L-Alanin an Position 33 gegen L-Valin,
b) Austausch des L-Alanin an Position 33 gegen L-Valin und Austausch des L-Serin an Position 211 gegen L-Phenylalanin,
enthält und die Aminosäuresequenz von SEQ ID NO:2 gegebenenfalls zusätzlich eine oder mehrere der Austausche, ausgewählt aus der Gruppe
c) Austausch des L-Lysin an Position 14 gegen eine Aminosäure ausgewählt aus der Gruppe L-Asparagin und L-Glutamin, bevorzugt L-Asparagin,
d) Austausch des L-Valin an Position 67 gegen eine Aminosäure ausgewählt aus der Gruppe L-Alanin, L-Lysin, L-Arginin und L-Histidin, bevorzugt L-Alanin,
e) Austausch des L-Threonin an Position 74 gegen eine Aminosäure ausgewählt aus der Gruppe L-Alanin, L-Lysin, L-Arginin und L-Histidin, bevorzugt L-Alanin,
f) Austausch des L-Glutaminsäure an Position 81 gegen eine Aminosäure ausgewählt aus der Gruppe L-Asparaginsäure, L-Serin, L-Alanin und L-Threonin, bevorzugt L-Asparaginsäure,
g) Austausch des L-Lysin an Position 84 gegen eine Aminosäure ausgewählt aus der Gruppe L-Glutamin, L-Serin, L-Threonin, L-Glutaminsäure, L-Asparaginsäure und L-Asparagin, bevorzugt L-Glutamin,
h) Austausch des L-Asparaginsäure an Position 85 gegen eine Aminosäure ausgewählt aus der Gruppe L-Alanin und Glycin, bevorzugt Glycin,
i) Austausch des L-Glutaminsäure an Position 238 gegen eine Aminosäure ausgewählt aus der Gruppe L-Asparaginsäure, L-Serin, L-Alanin und L-Threonin, bevorzugt L-Alanin,
j) Austausch des L-Lysin an Position 244 gegen eine Aminosäure ausgewählt aus der Gruppe L-Glutamin, L-Glutaminsäure, L-Asparaginsäure, L-Serin, L-Threonin und L-Asparagin, bevorzugt L-Glutamin,
k) Austausch des L-Asparagin an Position 254 gegen eine Aminosäure ausgewählt aus der Gruppe L-Threonin, L-Serin, L-Glutaminsäure und L-Asparaginsäure, bevorzugt L-Threonin,
l) Austausch des L-Prolin an Position 259 gegen eine Aminosäure ausgewählt aus der Gruppe L-Serin, L-Threonin, bevorzugt L-Threonin,
m) Austausch des L-Alanin an Position 260 gegen L-Prolin,
n) Austausch des L-Serin an Position 272 gegen eine Aminosäure ausgewählt aus der Gruppe L-Cystein und L-Methionin, bevorzugt L-Cystein,
o) Austausch des L-Lysin an Position 276 gegen eine Aminosäure ausgewählt aus der Gruppe L-Glutamin, L-Glutaminsäure, L-Asparaginsäure, L-Serin, L-Threonin und L-Asparagin, bevorzugt L-Glutamin,
p) Austausch der L-Asparaginsäure an Position 280 gegen eine Aminosäure ausgewählt aus der Gruppe L-Serin, L-Alanin, L-Glutaminsäure und L-Threonin, bevorzugt L-Glutaminsäure,
q) Austausch des L-Glutaminsäure an Position 313 gegen eine Aminosäure ausgewählt aus der Gruppe L-Serin, L-Alanin, L-Asparaginsäure und L-Threonin, bevorzugt L-Asparaginsäure,
r) Austausch des L-Glutaminsäure an Position 316 gegen eine Aminosäure ausgewählt aus der Gruppe L-Glutamin, L-Serin, L-Threonin, L-Asparaginsäure und L-Asparagin, bevorzugt L-Glutamin,
s) Austausch des L-Alanin an Position 319 gegen eine Aminosäure ausgewählt aus der Gruppe L-Threonin, L-Valin und L-Serin, bevorzugt L-Threonin,
t) Austausch des L-Alanin an Position 342 gegen eine Aminosäure ausgewählt aus der Gruppe L-Serin, L-Valin und L-Threonin, bevorzugt L-Serin oder L-Valin,
u) Austausch des L-Asparagin an Position 343 gegen eine Aminosäure ausgewählt aus der Gruppe L-Asparaginsäure, L-Glutaminsäure, L-Serin, L-Alanin und L-Threonin, bevorzugt L-Alanin.
enthält.

2. Nukleotidsequenz (DNA) gemäß Anspruch 1, **dadurch gekennzeichnet, dass** diese aus Mikroorganismen der Familie Enterobacteriaceae stammen.

3. Vektoren, **dadurch gekennzeichnet, dass** sie die Nukleotidsequenzen gemäß den Ansprüchen 1 oder 2 enthalten und gegebenenfalls in Mikroorganismen der Familie Enterobacteriaceae replizieren.

4. Mikroorganismen der Familie Enterobacteriaceae, **dadurch gekennzeichnet, dass** sie
a) die Nukleotidsequenzen gemäß den Ansprüchen 1 oder 2 enthalten und in denen die für die DAHP-Synthase kodierenden Nukleotidsequenzen exprimiert vorliegen.

5. Mikroorganismen gemäss Anspruch 4, **dadurch gekennzeichnet, dass** durch die Deletion oder Insertion von 1 bis 20 Aminosäuren in der Aminosäuresequenz von SEQ ID NO:2 die Konzentration oder Aktivität des aroG-Genproduktes (Proteins) nicht verändert sind, bezogen auf die Aktivität oder Konzentration des Genproduktes ohne Deletion oder Insertion von 1 bis 20 Aminosäuren.

6. Mikroorganismen gemäss den Ansprüchen 4 bis 5, **dadurch gekennzeichnet, dass** sie durch Transformation, Transduktion, Konjugation oder eine Kombination dieser Methoden hergestellt werden, mit einem Vektor, der mindestens eine der genannten für das Enzym DAHP-Synthase kodierenden, replizierbaren Nukleotidsequenzen, oder deren Allele oder Teile davon und/oder einen Promotor enthält.

7. Mikroorganismen gemäss Anspruch 6, **dadurch gekennzeichnet, dass** die Kopienzahl mindestens eines der genannten für das Enzym DAHP-Synthase kodierenden, replizierbaren Nukleotidsequenzen oder der Allele davon um mindestens 1 erhöht vorliegt.

8. Mikroorganismen gemäss Anspruch 7, **dadurch gekennzeichnet, dass** die Kopienzahl des aroG-Gens oder der Allele um höchstens 8, bevorzugt höchstens 4 erhöht vorliegt, oder
**dadurch gekennzeichnet, dass** die um mindestens 1 erhöhte Kopienzahl durch Integration des Gens oder der Allele in das Chromosom der Mikroorganismen oder durch einen extrachromosomal replizierenden Vektor erzielt wird.

9. Mikroorganismen gemäss den Ansprüchen 4 bis 8, **dadurch gekennzeichnet, dass** man
a) die Promotor- und Regulationsregion oder die Ribosomenbindungsstelle stromaufwärts des aroG-Gens mutiert, oder
b) Expresssionskassetten oder Promotoren stromaufwärts des aroG-Gens einbaut.

10. Mikroorganismen gemäss den Ansprüchen 4 bis 9, **dadurch gekennzeichnet, dass** die Expression des aroG-Gens oder Allels unter der Kontrolle eines die Expression des Gens verstärkenden Promotors steht.

11. Mikroorganismen gemäss den Ansprüchen 4 bis 10, **dadurch gekennzeichnet, dass** durch die Verstärkung des aroG-Gens die Konzentration oder Aktivität des aroG-Genproduktes (Proteins) um mindestens 10% erhöht sind, bezogen auf die Aktivität oder Konzentration des Genproduktes im für das aroG-Gen nicht rekombinanten Mikroorganismus oder Elternstamm.

12. Mikroorganismen gemäss den Ansprüchen 4 bis 11, **dadurch gekennzeichnet, dass** die Mikroorganismen aus den Gattungen Escherichia, Erwinia, Providencia und Serratia ausgewählt sind.

13. Mikroorganismen gemäss den Ansprüchen 4 bis 12, **dadurch gekennzeichnet, dass** sie organisch-chemische Verbindungen aus der Gruppe der Verbindungen des Shikimat-Stoffwechselweges produzieren,
vorzugsweise L-Tryptophan oder L-Phenylalanin.

14. Verfahren zur Herstellung von organisch-chemischen Verbindungen ausgewählt aus der Gruppe der Verbindungen des Shikimat-Stoffwechselweges, bevorzugt der aromatischen L-Aminosäuren, oder von die genannten Verbindungen enthaltenden Futtermitteladditiven, durch Fermentation von Mikroorganismen der Familie Enterobacteriaceae, **dadurch gekennzeichnet, dass** man
a) die die organisch-chemischen Verbindungen produzierenden Mikroorganismen gemäß den Ansprüchen 4 bis 13 in einem Medium kultiviert unter Bedingungen, bei denen die genannten Verbindungen im Medium oder in den Zellen angereichert werden, und
b) die genannten Verbindungen aus der Fermentationsbrühe isoliert, wobei ggf. weitere Bestandteile der Fermentationsbrühe und/oder die Biomasse in ihrer Gesamtheit oder Anteilen (≥ 0 bis 100%) im isolierten Produkt verbleiben oder vollständig entfernt werden.

15. Verfahren zur Herstellung von L-Tryptophan oder L-Tryptophan enthaltenden Futtermitteladditiven durch Fermentation von Mikroorganismen der Familie Enterobacteriaceae gemäß Anspruch 4, **dadurch gekennzeichnet, dass** man
a) die L-Tryptophan produzierenden Mikroorganismen gemäß den Ansprüchen 4 bis 13 in einem Medium kultiviert unter Bedingungen, bei denen L-Tryptophan im Medium oder in den Zellen angereichert wird, und
b) L-Tryptophan oder L-Tryptophan enthaltendes Futtermitteladditiv aus der Fermentationsbrühe isoliert, wobei ggf. weitere Bestandteile der Fermentationsbrühe und/oder die Biomasse in ihrer Gesamtheit oder Anteilen (≥ 0 bis 100%) im isolierten Produkt verbleiben oder vollständig entfernt werden.

16. Verfahren gemäss den Ansprüchen 14 und 15, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
• mindestens ein Gen des für die Anthranilat-Synthase, die Antranilat-Phosphoribosyltransferase, die Phosphoribosylanthranilat-Isomerase, die Indol-3-Glycerinphosphat-Synthase und die Tryptophan-Synthase kodierenden Tryptophan-Operons,
• das für die 3-Phosphoglycerat-Dehydrogenase kodierende serA-Gen,
• das für die Phosphoserinphosphatase kodierende serB-Gen,
• das für die Phosphoserinaminotransferase kodierende serC-Gen,
• das für die L-Tyrosin sensitive DHAP-Synthase kodierende aroF-Gen,
• das für die L-Tryptophan sensitive DHAP-Synthase kodierende aroH-Gen,
• das für die Phosphoenolpyruvat-Synthase kodierende pps-Gen,
• das für die Phosphoenolpyruvat-Carboxykinase kodierende pckA-Gen,
• das für die Transketolase A kodierende tktA-Gen,
• das für die Transketolase B kodierende tktB-Gen, und
• das Genprodukt des offenen Leserahmens (ORF) yddG von Escherichia coli
verstärkt, insbesondere überexprimiert.

17. Verfahren gemäss einem der Ansprüche 14 bis 16, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
• das für die Tryptophanase kodierende tnaA-Gen,
• das für den Repressor des trp-Operons kodierende trpR-Gen,
• das für die Chorismat-Mutase T und Prephenat-Dehydrogenase kodierende tyrA-Gen,
• das für die Chorismat-Mutase P und Prephenat-Dehydrogenase kodierende pheA-Gen,
• das für das Tryptophan spezifische Transportprotein kodierende mtr-Gen,
• das für die Tryptophan-Permease kodierende tnaB-Gen,
• das für den Transporter für aromatische Aminosäuren kodierende aroP-Gen,
• das für die L-Serin Deaminase kodierende sdaA-Gen,
• das für die Glukose-6-Phosphat-Isomerase kodierende pgi-Gen,
• das für die Tyrosin-Aminotransferase kodierende tyrB-Gen, und
• das für den Repressor des glp-Regulons kodierende glpR-Gen
abschwächt, gegebenenfalls ausschaltet oder die Expression verringert.

18. Verfahren gemäss Anspruch 14-17, **dadurch gekennzeichnet, dass** die Mikroorganismen in einem batch - Verfahren, einem fed batch - Verfahren, einem repeated fed batch-Verfahren oder einem kontinuierlichen Verfahren kultiviert werden
und/oder, dass die Konzentration der organisch-chemischen Verbindungen ausgewählt aus der Gruppe der Verbindungen des Shikimat-Stoffwechselweges zu einem oder mehreren Zeitpunkt(en) im Verlauf der Fermentation bestimmt wird.

## Claims

1. Nucleotide sequence (DNA) coding for a feedback-resistant variant of the enzyme 3-deoxy-D-*arabino*-heptulosonate-7-phosphate synthase (DAHP synthase), **characterized in that** its amino acid sequence has the amino acid sequence of SEQ ID NO:2, including deletion and/or insertion of from 1 to 20 amino acids, and essentially comprises a length of 350 amino acids, and contains within the corresponding amino acid sequences the amino acid substitutions in the amino acid sequence of SEQ ID NO:2, selected from the group consisting of
a) substitution of L-alanine at position 33 by L-valine,
b) substitution of L-alanine at position 33 by L-valine and substitution of L-serine at position 211 by L-phenylalanine,
and the amino acid sequence of SEQ ID NO:2, where appropriate, additionally contains one or more of the substitutions selected from the group consisting of
c) substitution of L-lysine at position 14 by an amino acid selected from the group consisting of L-asparagine and L-glutamine, preferably L-asparagine,
d) substitution of L-valine at position 67 by an amino acid selected from the group consisting of L-alanine, L-lysine, L-arginine and L-histidine, preferably L-alanine,
e) substitution of L-threonine at position 74 by an amino acid selected from the group consisting of L-alanine, L-lysine, L-arginine and L-histidine, preferably L-alanine,
f) substitution of L-glutamic acid at position 81 by an amino acid selected from the group consisting of L-aspartic acid, L-serine, L-alanine and L-threonine, preferably L-aspartic acid,
g) substitution of L-lysine at position 84 by an amino acid selected from the group consisting of L-glutamine, L-serine, L-threonine, L-glutamic acid, L-aspartic acid and L-asparagine, preferably L-glutamine,
h) substitution of L-aspartic acid at position 85 by an amino acid selected from the group consisting of L-alanine and glycine, preferably glycine,
i) substitution of L-glutamic acid at position 238 by an amino acid selected from the group consisting of L-aspartic acid, L-serine, L-alanine and L-threonine, preferably L-alanine,
j) substitution of L-lysine at position 244 by an amino acid selected from the group consisting of L-glutamine, L-glutamic acid, L-aspartic acid, L-serine, L-threonine and L-asparagine, preferably L-glutamine,
k) substitution of L-asparagine at position 254 by an amino acid selected from the group consisting of L-threonine, L-serine, L-glutamic acid and L-aspartic acid, preferably L-threonine,
1) substitution of L-proline at position 259 by an amino acid selected from the group consisting of L-serine, L-threonine, preferably L-threonine,
m) substitution of L-alanine at position 260 by L-proline,
n) substitution of L-serine at position 272 by an amino acid selected from the group consisting of L-cysteine and L-methionine, preferably L-cysteine,
o) substitution of L-lysine at position 276 by an amino acid selected from the group consisting of L-glutamine, L-glutamic acid, L-aspartic acid, L-serine, L-threonine and L-asparagine, preferably L-glutamine,
p) substitution of L-aspartic acid at position 280 by an amino acid selected from the group consisting of L-serine, L-alanine, L-glutamic acid and L-threonine, preferably L-glutamic acid,
q) substitution of L-glutamic acid at position 313 by an amino acid selected from the group consisting of L-serine, L-alanine, L-aspartic acid and L-threonine, preferably L-aspartic acid,
r) substitution of L-glutamic acid at position 316 by an amino acid selected from the group consisting of L-glutamine, L-serine, L-threonine, L-aspartic acid and L-asparagine, preferably L-glutamine,
s) substitution of L-alanine at position 319 by an amino acid selected from the group consisting of L-threonine, L-valine and L-serine, preferably L-threonine,
t) substitution of L-alanine at position 342 by an amino acid selected from the group consisting of L-serine, L-valine and L-threonine, preferably L-serine or L-valine,
u) substitution of L-asparagine at position 343 by an amino acid selected from the group consisting of L-aspartic acid, L-glutamic acid, L-serine, L-alanine and L-threonine, preferably L-alanine.

2. Nucleotide sequence (DNA) according to Claim 1, **characterized in that** it is derived from microorganisms of the *Enterobacteriaceae* family.

3. Vectors, **characterized in that** they include the nucleotide sequences according to either of Claims 1 and 2, and optionally replicate in microorganisms of the *Enterobacteriaceae* family.

4. Microorganisms of the *Enterobacteriaceae* family, **characterized in that** they
a) harbour the nucleotide sequences according to either of Claims 1 and 2, and in which the expressed nucleotide sequences coding for DAHP synthase are present.

5. Microorganisms according to Claim 4, **characterized in that** deletion or insertion of from 1 to 20 amino acids in the amino acid sequence of SEQ ID NO:2 does not alter the concentration or activity of the aroG gene product (protein), based on the activity or concentration of the gene product without deletion or insertion of from 1 to 20 amino acids.

6. Microorganisms according to Claims 4 and 5, **characterized in that** they are produced by transformation, transduction, conjugation, or any combination of these methods, with a vector which includes at least one of said replicable nucleotide sequences coding for the enzyme DAHP synthase, or their alleles or parts thereof, and/or a promoter.

7. Microorganisms according to Claim 6, **characterized in that** the copy number of at least one of said replicable nucleotide sequences coding for the enzyme DAHP synthase, or of the alleles thereof has increased by at least 1.

8. Microorganisms according to Claim 7, **characterized in that** the copy number of the aroG gene or of the alleles has increased by no more than 8, preferably no more than 4, or **characterized in that** the copy number is increased by at least 1 by integrating the gene or the alleles into the chromosome of the microorganisms or by a vector replicating extra-chromosomally.

9. Microorganisms according to Claims 4 to 8, **characterized in that**
a) the promoter and regulatory regions or the ribosome binding site upstream of the aroG gene are mutated, or
b) expression cassettes or promoters are incorporated upstream of the aroG gene.

10. Microorganisms according to Claims 4 to 9, **characterized in that** expression of the aroG gene or allele is under the control of a promoter enhancing expression of said gene.

11. Microorganisms according to Claims 4 to 10, **characterized in that** enhancing the aroG gene increases the concentration or activity of the aroG gene product (protein) by at least 10%, based on the activity or concentration of the gene product in the microorganism or parent strain that is non-recombinant for the aroG gene.

12. Microorganisms according to Claims 4 to 11, **characterized in that** the microorganisms are selected from the genera *Escherichia, Erwinia, Providencia* and *Serratia.*

13. Microorganisms according to Claims 4 to 12, **characterized in that** they produce organochemical compounds from the group of compounds of the shikimate metabolic pathway, preferably L-tryptophan or L-phenylalanine.

14. Method for manufacturing organochemical compounds selected from the group of compounds of the shikimate metabolic pathway, preferably the aromatic L-amino acids, or feed additives containing said compounds, by fermentation of microorganisms of the *Enterobacteriaceae* family, **characterized in that**
a) the microorganisms according to Claims 4 to 13 that produce the organochemical compounds are cultured in a medium under conditions in which said compounds are accumulated in the medium or in the cells, and
b) said compounds are isolated from the fermentation broth, optionally with further components of the fermentation broth and/or all or parts (≥ 0 to 100%) of the biomass remaining in the isolated product or being removed completely.

15. Method for manufacturing L-tryptophan or L-tryptophan-containing feed additives by fermentation of microorganisms of the *Enterobacteriaceae* family according to Claim 4, **characterized in that**
a) the L-tryptophan-producing microorganisms according to Claims 4 to 13 are cultured in a medium under conditions in which L-tryptophan is accumulated in the medium or in the cells, and
b) L-tryptophan or L-tryptophan-containing feed additive is isolated from the fermentation broth, optionally with further components of the fermentation broth and/or all or parts (≥ 0 to 100%) of the biomass remaining in the isolated product or being removed completely.

16. Method according to Claims 14 and 15, **characterized in that** L-tryptophan is manufactured by fermenting microorganisms of the *Enterobacteriaceae* family, in which additionally one or more of the genes selected from the group consisting of:
• at least one gene of the tryptophan operon coding for anthranilate synthase, anthranilate phosphoribosyltransferase, phosphoribosyl anthranilate isomerase, indole-3-glycerol-phosphate synthase and tryptophan synthase,
• the serA gene coding for 3-phosphoglycerate dehydrogenase,
• the serB gene coding for phosphoserine phosphatase,
• the serC gene coding for phosphoserine aminotransferase,
• the aroF gene coding for L-tyrosine-sensitive DHAP synthase,
• the aroH gene coding for L-tryptophan-sensitive DHAP synthase,
• the pps gene coding for phosphoenolpyruvate synthase,
• the pckA gene coding for phosphoenolpyruvate carboxykinase,
• the tktA gene coding for transketolase A,
• the tktB gene coding for transketolase B, and
• the gene product of the *Escherichia coli* open reading frame (ORF) yddG,
is/are simultaneously enhanced, more specifically overexpressed.

17. Method according to any of Claims 14 to 16, **characterized in that** L-tryptophan is manufactured by fermenting microorganisms of the *Enterobacteriaceae* family, in which additionally one or more of the genes selected from the group consisting of:
• the tnaA gene coding for tryptophanase,
• the trpR gene coding for the trp operon repressor,
• the tyrA gene coding for chorismate mutase T and prephenate dehydrogenase,
• the pheA gene coding for chorismate mutase P and prephenate dehydrogenase,
• the mtr gene coding for the tryptophan-specific transport protein,
• the tnaB gene coding for tryptophan permease,
• the aroP gene coding for the transporter for aromatic amino acids,
• the sdaA gene coding for L-serine deaminase,
• the pgi gene coding for glucose-6-phosphate isomerase,
• the tyrB gene coding for tyrosine aminotransferase, and
• the glpR gene coding for the glp regulon repressor,
is/are simultaneously attenuated, where appropriate eliminated, or expression is reduced.

18. Method according to Claims 14-17, **characterized in that** the microorganisms are cultured in a batch process, a fed-batch process, a repeated fed-batch process, or a continuous process, and/or that the concentration of the organochemical compounds selected from the group of compounds of the shikimate metabolic pathway is determined at one or more point (s) in the course of the fermentation.

## Revendications

1. Séquence nucléotidique (ADN) qui code pour une variante résistante à la rétroaction de l'enzyme 3-désoxy-D-arabino-heptulosonate-7-phosphate-synthase (DAHP-synthase), **caractérisée en ce que** sa séquence d'acides aminés comprend la séquence d'acides aminés SEQ ID NO:2, y compris la délétion et/ou l'insertion de 1 à 20 acides aminés, et pour l'essentiel présente une longueur de 350 acides aminés et, dans les séquences d'acides aminés correspondantes, contient les échanges d'acides aminés dans la séquence d'acides aminés SEQ ID NO:2 choisis dans le groupe suivant :
a) remplacement de la L-alanine en position 33 par une L-valine,
b) remplacement de la L-alanine en position 33 par une L-valine et remplacement de la L-sérine en position 211 par une L-phénylalanine,
et la séquence d'acides aminés SEQ ID NO:2 contient éventuellement en outre un ou plusieurs des échanges choisis dans le groupe consistant en :
c) le remplacement de la L-lysine en position 14 par un acide aminé choisi dans le groupe consistant en la L-asparagine et la L-glutamine, de préférence la L-asparagine,
d) le remplacement de la L-valine en position 67 par un acide aminé choisi dans le groupe consistant en la L-alanine, la L-lysine, la L-arginine et la L-histidine, de préférence la L-alanine,
e) le remplacement de la L-thréonine en position 74 par un acide aminé choisi dans le groupe consistant en la L-alanine, la L-lysine, la L-arginine et la L-histidine, en particulier la L-alanine,
f) le remplacement de l'acide L-glutamique en position 81 par un acide aminé choisi dans le groupe consistant en l'acide L-asparagique, la L-sérine, la L-alanine et la L-thréonine, de préférence l'acide L-asparagique,
g) le remplacement de la L-lysine en position 84 par un acide aminé choisi dans le groupe consistant en la L-glutamine, la L-sérine, la L-thréonine, l'acide L-glutamique, l'acide L-asparagique et la L-asparagine, de préférence la L-glutamine,
h) le remplacement de l'acide L-asparagique en position 85 par un acide aminé choisi dans le groupe consistant en la L-alanine et la glycine, de préférence la glycine,
i) le remplacement de l'acide L-glutamique en position 238 par un acide aminé choisi dans le groupe consistant en l'acide L-asparagique, la L-sérine, la L-alanine et la L-thréonine, de préférence la L-alanine,
j) le remplacement de la L-lysine en position 244 par un acide aminé choisi dans le groupe consistant en la L-glutamine, l'acide L-glutamique, l'acide L-asparagique, la L-sérine, la L-thréonine et la L-asparagine, de préférence la L-glutamine,
k) le remplacement de la L-asparagine en position 254 par un acide aminé choisi dans le groupe consistant en la L-thréonine, la L-sérine, l'acide L-glutamique et l'acide L-asparagique, de préférence la L-thréonine,
1) le remplacement de la L-proline en position 259 par un acide aminé choisi dans le groupe consistant en la L-sérine, la L-thréonine, de préférence la L-thréonine,
m) le remplacement de la L-alanine en position 260 par une L-proline,
n) le remplacement de la L-sérine en position 272 par un acide aminé choisi dans le groupe consistant en la L-cystéine et la L-méthionine, de préférence la L-cystéine,
o) le remplacement de la L-lysine en position 276 par un acide aminé choisi dans le groupe consistant en la L-glutamine, l'acide L-glutamique, l'acide L-asparagique, la L-sérine, la L-thréonine et la L-asparagine, de préférence la L-glutamine,
p) le remplacement de l'acide L-asparagique en position 280 par un acide aminé choisi dans le groupe consistant en la L-sérine, la L-alanine, l'acide L-glutamique et la L-thréonine, de préférence l'acide L-glutamique,
q) le remplacement de l'acide L-glutamique en position 313 par un acide aminé choisi dans le groupe consistant en la L-sérine, la L-alanine, l'acide L-asparagique et la L-thréonine, de préférence l'acide L-asparagique,
r) le remplacement de l'acide L-glutamique en position 316 par un acide aminé choisi dans le groupe consistant en la L-glutamine, la L-sérine, la L-thréonine, l'acide L-asparagique et la L-asparagine, de préférence la L-glutamine,
s) le remplacement de la L-alanine en position 319 par un acide aminé choisi dans le groupe consistant en la L-thréonine, la L-valine et la L-sérine, de préférence la L-thréonine,
t) le remplacement de la L-alanine en position 342 par un acide aminé choisi dans le groupe consistant en la L-sérine, la L-valine et la L-thréonine, de préférence la L-sérine ou la L-valine,
u) le remplacement de la L-asparagine en position 343 par un acide aminé choisi dans le groupe consistant en l'acide L-asparagique, l'acide L-glutamique, la L-sérine, la L-alanine et la L-thréonine, de préférence la L-alanine.

2. Séquence nucléotidique (ADN) selon la revendication 1, **caractérisée en ce qu'**elle dérive de microorganismes de la famille des Enterobacteriaceae.

3. Vecteurs, **caractérisés en ce qu'**ils contiennent les séquences nucléotidiques selon les revendications 1 ou 2 et éventuellement se répliquent dans des microorganismes de la famille des Enterobacteriaceae.

4. Microorganismes de la famille des Enterobacteriaceae, **caractérisés en ce que**
a) ils contiennent les séquences nucléotidiques selon les revendications 1 ou 2, et les séquences nucléotidiques codant pour la DAHP-synthase s'y présentent sous forme exprimée.

5. Microorganismes selon la revendication 4, **caractérisés en ce que** la délétion ou l'insertion de 1 à 20 acides aminés dans la séquence d'acides aminés SEQ ID N0:2 ne modifie pas la concentration ou l'activité du produit génique aroG (protéines), par rapport à l'activité ou à la concentration du produit génique sans délétion ni insertion de 1 à 20 acides aminés.

6. Microorganismes selon les revendications 4 à 5, **caractérisés en ce qu'**ils sont fabriqués par transformation, transduction, conjugaison, ou par une combinaison de ces méthodes, avec un vecteur qui contient au moins l'une des séquences nucléotidiques réplicables mentionnées, codant pour l'enzyme DAHP-synthase, ou leurs allèles ou parties, et/ou un promoteur.

7. Microorganismes selon la revendication 6, **caractérisés en ce que** le nombre de copies d'au moins l'une des séquences nucléotidiques réplicables mentionnées, codant pour l'enzyme DAHP-synthase, ou leurs allèles, est augmenté d'au moins 1.

8. Microorganismes selon la revendication 7, **caractérisés en ce que** le nombre de copies du gène aroG ou des allèles est augmenté d'au plus 8, de préférence d'au plus 4, ou
**caractérisé en ce que** le nombre de copies augmenté d'au moins 1 est réalisé par intégration du gène ou des allèles dans le chromosome des microorganismes, ou grâce à un vecteur de réplication extrachromosomique.

9. Microorganismes selon les revendications 4 à 8, **caractérisés en ce que**
a) on mute la région promotrice ou régulatrice, ou le site de fixation du ribosome, en amont du gène aroG, ou
b) on incorpore des cassettes d'expression ou des promoteurs en amont du gène aroG.

10. Microorganismes selon les revendications 4 à 9, **caractérisés en ce que** l'expression du gène aroG ou de l'allèle est sous le contrôle d'un promoteur renforçant l'expression du gène.

11. Microorganismes selon les revendications 4 à 10, **caractérisés en ce que** le renforcement du gène aroG augmente la concentration ou l'activité du produit génique aroG (protéines) d'au moins 10 %, par rapport à l'activité ou à la concentration du produit génique dans le microorganisme ou la souche parente non recombinant pour le gène aroG.

12. Microorganismes selon les revendications 4 à 11, **caractérisés en ce que** les microorganismes sont choisis parmi les genres Escherichia, Erwinia, Providencia et Serratia.

13. Microorganismes selon les revendications 4 à 12, **caractérisés en ce qu'**ils produisent des composés de la chimie organique du groupe des composés de la voie métabolique du shikimate, de préférence le L-tryptophane ou la L-phénylalanine.

14. Procédé de préparation de composés de la chimie organique, choisis dans le groupe des composés de la voie métabolique du shikimate, de préférence les acides L-aminés aromatiques, ou d'additifs pour l'alimentation animale contenant les composés mentionnés, par fermentation de microorganismes de la famille des Enterobacteriaceae, **caractérisé en ce que**
a) on cultive dans un milieu les microorganismes produisant les composés de la chimie organique selon les revendications 4 à 13 dans les conditions dans lesquelles les composés mentionnés sont enrichis dans le milieu ou dans les cellules, et
b) on isole du bouillon de fermentation les composés mentionnés, auquel cas éventuellement d'autres constituants du bouillon de fermentation et/ou la biomasse restent en totalité ou en partie (≥ 0 à 100 %) dans le produit isolé, ou sont entièrement éliminés.

15. Procédé de préparation de L-tryptophane ou d'additifs pour l'alimentation animale contenant du L-tryptophane, par fermentation de microorganismes de la famille des Enterobacteriaceae selon la revendication 4, **caractérisé en ce que**
a) on cultive dans un milieu les microorganismes produisant le L-tryptophane selon les revendications 4 à 13, dans les conditions dans lesquelles le L-tryptophane est enrichi dans le milieu ou dans les cellules, et
b) on isole du bouillon de fermentation le L-tryptophane ou l'additif pour l'alimentation animale contenant du L-tryptophane, auquel cas éventuellement d'autres constituants du bouillon de fermentation, et/ou la biomasse, restent en totalité ou en partie (≥ 0 à 100 %) dans le produit isolé, ou sont entièrement éliminés.

16. Procédé selon les revendications 14 et 15, **caractérisé en ce que**, pour préparer du L-tryptophane, on fermente des microorganismes de la famille des Enterobacteriaceae, dans lesquels on renforce, éventuellement on surexprime, simultanément un ou plusieurs gènes choisis dans le groupe consistant en :
• au moins un gène de l'opéron tryptophane codant pour l'anthranilate-synthase, pour l'anthranilate-phosphoribosyltransférase, la phosphoribosylanthranilate-isomérase, l'indole-3-glycérolphosphate-synthase et la tryptophane-synthase,
• le gène serA, codant pour la 3-phosphoglycérate-déshydrogénase,
• le gène serB, codant pour la phosphosérinephosphatase,
• le gène serC, codant pour la phosphosérineaminotransférase,
• le gène aroF, codant pour la DHAP-synthase sensible à la L-tyrosine,
• le gène aroH, codant pour la DHAP-synthase sensible au L-tryptophane,
• le gène pps, codant pour la phosphoénolpyruvate-synthase,
• le gène pckA, codant pour la phosphoénolpyruvate-carboxykinase,
• le gène tktA, codant pour la transcétolase A,
• le gène tktB, codant pour la transcétolase B, et
• le produit génique du cadre ouvert de lecture (ORF) yddG d'Escherichia coli.

17. Procédé selon l'une des revendications 14 à 16, **caractérisé en ce que**, pour la préparation du L-tryptophane, on fermente des microorganismes de la famille des Enterobacteriaceae, dans lesquels, en outre et simultanément, on atténue, éventuellement on inactive un ou plusieurs des gènes choisis dans le groupe consistant en :
• le gène tnaA, codant pour la tryptophanase,
• le gène trpR, codant pour le répresseur de l'opéron trp,
• le gène tyrA, codant pour la chorismate-mutase T et la préphénate-déshydrogénase,
• le gène pheA, codant pour la chorismate-mutase P et la préphénate-déshydrogénase,
• le gène mtr, codant pour la protéine de transport spécifique du tryptophane,
• le gène tnaB, codant pour la tryptophane-perméase,
• le gène aroP, codant pour le transporteur des acides aminés aromatiques,
• le gène sdaA, codant pour la L-sérine désaminase,
• le gène pgi, codant pour la glucose-6-phosphate-isomérase,
• le gène tyrB, codant pour la tyrosine-aminotransférase, et
• le gène glpR, codant pour le répresseur du régulon glp,
ou on en diminue l'expression.

18. Procédé selon les revendications 14 à 17, **caractérisé en ce qu'**on cultive les microorganismes dans le cadre d'un procédé discontinu, d'un procédé discontinu alimenté, d'un procédé discontinu alimenté répété ou d'un procédé continu,
et/ou **en ce que** la concentration des composés de la chimie organique, choisis dans le groupe des composés de la voie du métabolisme du shikimate, est déterminée à un ou plusieurs instants au cours de la fermentation.
